# EUROPEAN PATENT SPECIFICATION

(11) **EP 1 363 935 B1**
(45) Date of publication and mention of the grant of the patent: **14.11.2012**
(21) Application number: 02718911.7
(22) Date of filing: 07.02.2002
(51) Int. Cl.: C12N 15/11, C12N 15/64, C12N 15/66, C12N 15/74, C12P 19/34, C12Q 1/68

(54) **TER SITES AND TER BINDING PROTEINS**
TER-STELLEN UND TER-BINDENDE PROTEINE
SITES TER ET PROTÉINES DE LIAISON AU SITE TER.

(30) Priority: 07.02.2001 US 266846 P
(43) Date of publication of application: 26.11.2003
(73) Proprietor: Life Technologies Corporation, Carlsbad, CA 92008 (US)
(72) Inventor: BYRD, Devon, Fredericksburg, VA 22405 (US); HARTLEY, James, Frederick, MD 21702 (US); YOUNG, Alice, Gaithersburg, MD 20878 (US)
(74) Representative: Harrison Goddard Foote
(86) International application number: PCT/US2002/003366
(87) International publication number: WO 2002/062957

(56) References cited:
- SMITH ET AL.: "The minimal sequence needed to define a functional DNA terminator in Bacillus subtilis" JOURNAL OF MOLECULAR BIOLOGY, vol. 241, - 1994 pages 335-340, XP002279188
- DUGGIN ET AL.: 'Site-directed mutants of RTP of bacillus subtilis and the mechanism of replication fork arrest' JOURNAL OF MOLECULAR BIOLOGY vol. 286, 1999, pages 1325 - 1335, XP002951704
- HIDAKA ET AL.: 'Purification of a DNA replication terminus (ter) site-binding protein in escherichia coli and identification of the structural gene' THE JOURNAL OF BIOLOGICAL CHEMISTRY vol. 264, no. 35, 15 December 1989, pages 21031 - 21037, XP002951705
- HILL ET AL.: 'Identification of the DNA sequence from the E. coli terminus region that halts replication forks' CELL vol. 55, 04 November 1988, pages 459 - 466, XP002951706
- NEYLON ET AL.: 'Interaction of the escherichia coli replication terminator protein (Tus) with DNA: a model derived from DNA-binding studies of mutant proteins by surface plasmon resonance' BIOCHEMISTRY vol. 39, 2000, pages 11989 - 11999, XP002951707
- BUSSIERE ET AL.: 'Terminaton of DNA replication of bacterial and plasmid chromosomes' MOLECULAR MICROBIOLOGY vol. 31, no. 6, 1999, pages 1611 - 1618, XP002951708
- GRIFFITHS ET AL.: 'Replication terminator protein-based replication fork-arrest systems in various bacillus species' JOURNAL OF BACTERIOLOGY vol. 180, no. 13, July 1998, pages 3360 - 3367, XP002951709

## Description

### BACKGROUND OF THE INVENTION

### Field of the Invention

The present invention is in the field of molecular biology. The invention is related generally to polynucleotide and polypeptides that interact specifically with the polynucleotides, and methods for their use. Specifically, the invention provides solid supports comprising at least one nucleic acid molecule that comprises all or a portion of a Ter site; a method for attaching a Ter-binding protein and/or fusion protein comprising a Ter-binding protein to a solid support; and a method for attaching a nucleic acid to a said support.

### Related Art

In bacterial systems, replication of genomes and plasmids begins at a specific site on the genome or plasmid termed the origin of replication (ori). Replication is initiated at the origin of replication and proceeds either unidirectionally or bidirectionally from the origin to a defined sequence located at an appropriate part (appropriate for the specific replicon) of the genome or plasmid called a termination sequence (Ter-site) where the replication complex is halted and replication terminated.

In order to correctly terminate replication at a Ter-site, an organism must express a functional replication terminator protein (RTP). RTPs are nucleic acid binding proteins which bind to the Ter-sites and form an RTP-Tcr complex. The bound RTPs are believed to function in replication termination by preventing the helicase activity of the replication complex from unwinding the Ter-site. This activity is termed a contrahelicase activity. RTPs and Ter-sites have been identified in a wide variety of Gram positive and Gram negative microorganisms including, for example, *Bacillus subtilis* and *Escherichia coli.*(*See* Bussiere, et al., Mol. Micro. 31(6):1611-1618 (1999) and Griffiths, et al., J. Bacteriology 180(13):3360-3367 (1998)).

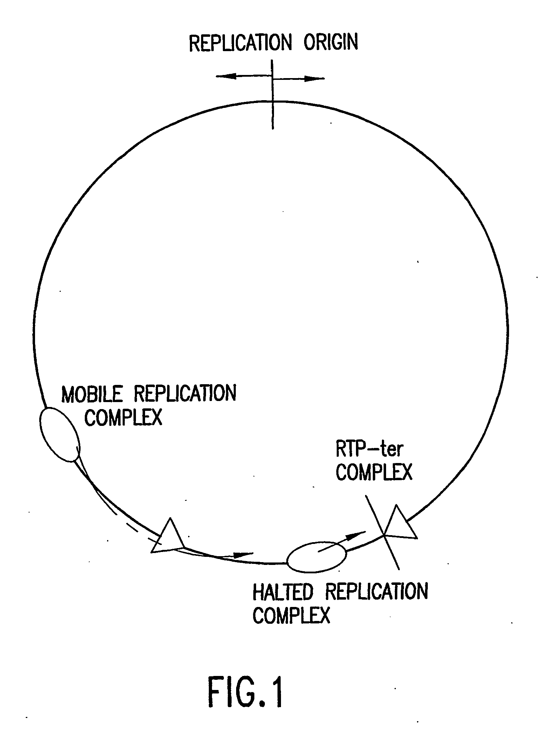

The ability of most RTP-Ter complexes to halt replication is unidirectional; a replication complex approaching from one direction the non-permissive direction-would be halted while one approaching from the opposite direction-the permissive direction-would be allowed to pass. With some modified RTPs the ability to halt replication is bi-directional and these RTPs can halt replication from either direction. Under normal-unidirectional conditions, to achieve correct termination of replication, there are generally at least two Ter-sites located on each genome or plasmid. The Ter-sites are arranged so as to permit passage of a replication fork into the region between the Ter-sitcs from either direction but prevent exit of the replication fork from the region. A replication complex will pass through a first Ter-site and be stopped at a second Ter-site while a replication complex approaching from the opposite direction will pass through the second site and be stopped at the first. This is shown schematically in Fig. 1.

RTPs have been found to bind Ter-sites extremely tightly, resulting in very stable RTP-Ter complexes with long half lives. The high affinity of RTPs for Ter-sites and the directionality of the Ter-sites can be exploited for use in the methods and kits described in the present invention.

### SUMMARY OF THE INVENTION

The present invention provides a solid support comprising:
at least one nucleic acid molecule that comprises all or a portion of a Ter site, wherein the nucleic acid molecule is directly attached to the support; and
at least one fusion protein having one or more Ter-binding portions and one or more additional polypeptide portions;
wherein the fusion protein is indirectly attached to the support via attachment to the nucleic acid molecule through interaction between the nucleic acid molecule Ter site or portion thereof and the fusion protein Ter-binding portion.

The invention also provides a method for attaching a nucleic acid to a solid support, comprising:
attaching one or more Ter-binding proteins to a solid support; and
contacting the Ter-binding protein with a first nucleic acid, said nucleic acid comprising a Ter-site.

### SUMMARY OF THE INVENTION

The present invention provides materials and methods especially useful in molecular biology applications. Generally, the disclosure describes use of one or more Ter-sites and/or one or more Ter-binding proteins or RTPs *in vitro* (e.g., outside a cell), *in vivo* (e.g., within a cell), or combinations thereof. In one embodiment, the present disclosure describes one or more nucleic acid molecules (which may be isolated) comprising at least one Ter-site and/or portions thereof. Such nucleic acid molecules may be any form or type of nucleic acid molecule such as linear, circular, supercoiled, single stranded, double stranded, double stranded with one or more single stranded regions (e.g., at least one single stranded overhang at one or more termini of the molecules), etc. and may be isolated or contained by one or more hosts or host cells. Preferred nucleic acid molecules include vectors, integration sequences (e.g., transposons), plasmids, cosmids, artificial chromosomes (e.g., BACs and YACs), phagemids and the like. Such Ter-sites and/or portions thereof may be located at any position and in any orientation in the nucleic acid molecules including one or more positions within the molecules and/or at or near one or more termini of such molecules. In some embodiments, the nucleic acid molecules may optionally comprise one or more detectable atoms or groups, for example, one or more radioisotopes, chromophorcs, fluorophores, enzymes, epitopes, haptens, antigens and/or combinations thereof. In one aspect, the nucleic acid molecules may be bound to one or more Ter-binding protein.

The present disclosure also describes compositions or reaction mixtures comprising one or more of the nucleic acid molecules. Such compositions or reaction mixtures may also comprise one or more other components for carrying out the methods described. Such other components may include one or more Ter-binding proteins which may be bound and/or unbound to such one or more Ter-sites or portions thereof, one or more ligases, one or more polymerases, one or more topoisomerases, one or more recombination proteins, one or more host cells (which may be competent to take up nucleic acid molecules), one or more solid supports (which may have one or more Ter-binding proteins and/or nucleic acid molecules comprising one or more Ter-sites or portions thereof bound directly or indirectly to such support, and the like.

In another aspect, the present disclosure describes a modified protein comprising a Ter-binding protein and one or more modifications. In some aspects, the modifying group may be chemically attached to the Ter-binding protein. In some embodiments, the modification may be to create a fusion protein comprising a Ter-binding portion and one or more additional polypeptide portions. The additional polypeptide portions maybe one or more enzymes, binding polypeptides, i.e., antibodies, Fabs and the like, epitopes, antigens, haptcns and the like and combinations thereof. Fusion proteins may optionally comprise a linker between two portions, for example, between a Ter-binding portion and an enzyme portion. A linker may optionally comprise one or more cleavage sites, for example, a cleavage site for one or more proleolytic enzymes and/or one or more sites susceptible to chemical cleavage.

The present disclosure describes solid supports to which are attached, covalently or non-covalently, nucleic acids and/or proteins as described herein. In some embodiments, the solid supports of the present invention may comprise at least one oligonucleotide comprising all or a portion of one or more Ter sites. In some embodiments, the oligonucleotide may be in the form of a hairpin or stem-loop. In some embodiments, the solid supports of the present invention may comprise all or a portion or one or more Ter-binding proteins. In another aspect, the present disclosure describes compositions comprising solid supports of the present invention.

More specifically, the present disclosure describes the use of at least one Ter sequence in one or more nucleic acid molecules for use in *in vitro* and/or *in vivo* cloning (preferably directional cloning). This allows for positive selection for nucleic acid molecules of interest (preferably those that have been cloned in a desired orientation).

The present disclosure describes a method of cloning by providing at least one nucleic acid molecule of the invention comprising all or a portion of a Ter-site and at least one vector, inserting or cloning all or a portion of said at least one nucleic acid molecule into said at least one vector, and selecting at least one vector comprising all or a portion of said at least one nucleic acid molecule in the desired orientation.

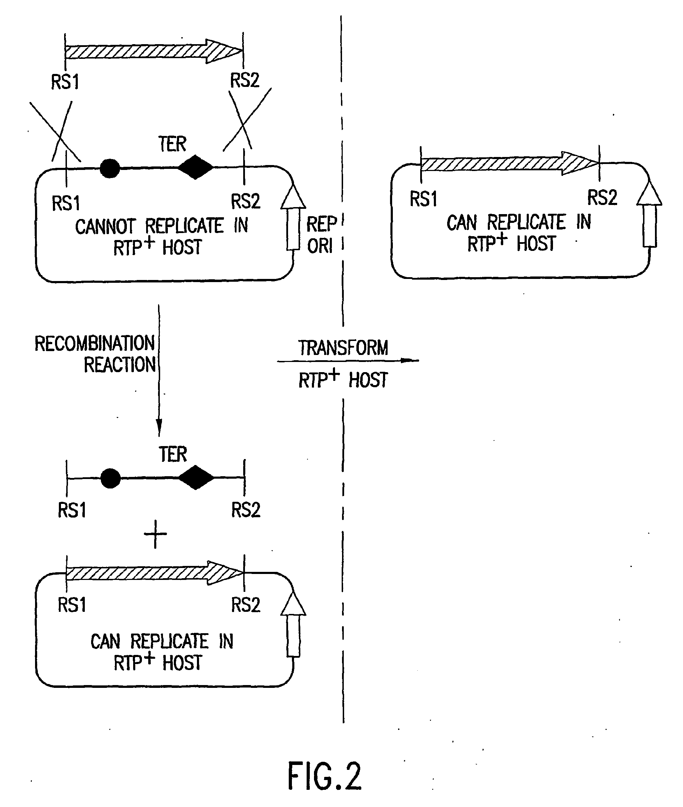

The present disclosure describes a method of cloning by providing at least one vector comprising all or a portion of at least one Ter-site and at least one nucleic acid molecule, inserting or cloning all or a portion of the at least one nucleic acid molecule into the at least one vector, and selecting at least one vector comprising all or a portion of the at least one nucleic acid molecule, preferably in the desired orientation (Fig. 2).

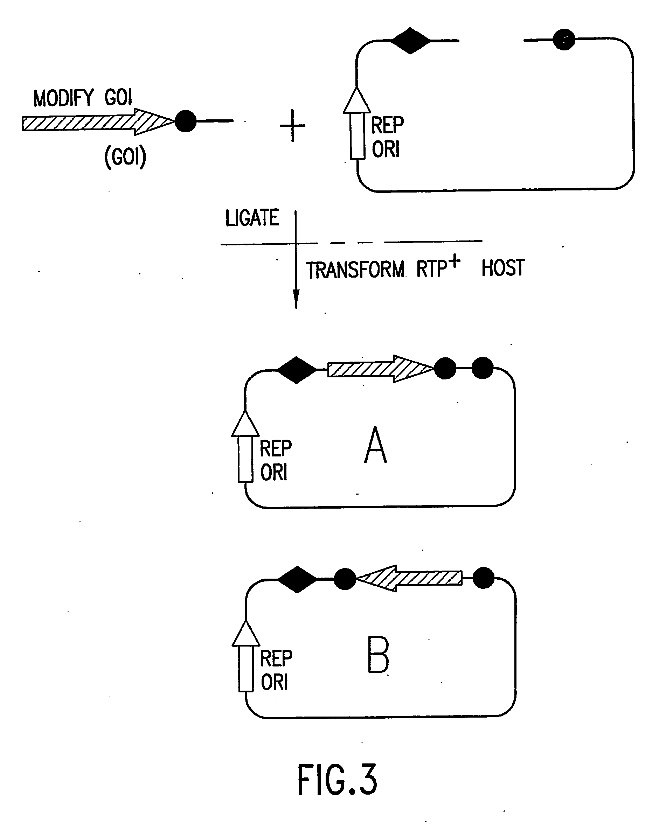

The present disclosure describes a method of cloning by providing at least one nucleic acid molecule of interest comprising all or a portion of at least one Ter-site, providing at least one vector comprising all or a portion of at least one Ter-site, inserting or cloning all or a portion of the at least one nucleic acid molecule into the at least one vector, and selecting at least one vector comprising all or a portion of the at least one nucleic acid molecule in the desired orientation (Fig. 3).

In some embodiments, the methods described may also comprise selecting against undesired nucleic acid molecules (including vectors). Such selections may involve selecting against molecules having all or a portion of a Ter-site in a selectable conformation or orientation and/or selecting for molecules having all or a portion of a Ter-site in a selectable conformation or orientation. In some embodiments, the selecting step comprises introducing (e.g., by transformation or transfection) the vector molecule into a host cell, wherein the host cell expresses at least one Ter-binding protein.

Thus, the present disclosure describes a method of directional insertion or cloning of nucleic acid molecules using one or more Ter sequences or portions thereof. In some embodiments, the desired orientation of the nucleic acid molecule in the vector is the orientation in which the Ter-site in the nucleic acid molecule permits replication in the same direction as the Ter-site in the vector. In this embodiment, at least one Ter-site prevents replication of the vector when the nucleic acid molecule is in the undesired orientation (Fig. 3). In another embodiment, the desired orientation of the nucleic acid molecule in the vector avoids generation of a functional Ter-site. In the undesired orientation, at least one functional Ter-site is generated which prevents replication of the vector. Thus, for example, when the Ter-site in the nucleic acid molecule and the Ter-site in the vector arc partial Ter-sites, insertion of the nucleic acid molecule may or may not generate a functional Ter-site, depending, e.g., on the orientation. In this case, the desired orientation will not generate a functional Ter-site thus allowing replication of the recombinant vector.

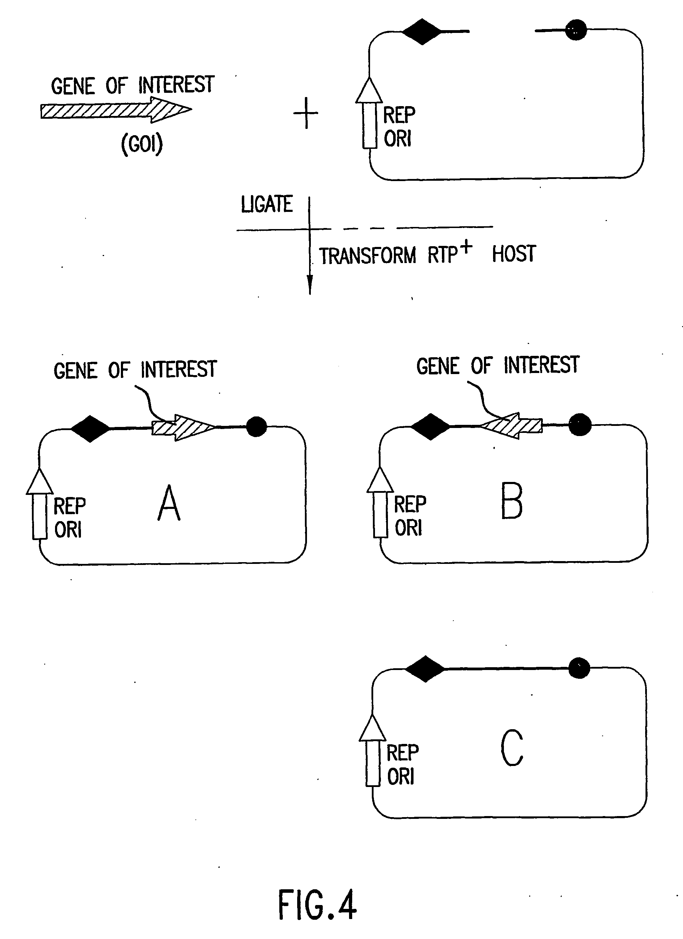

The present disclosure describes the use of at least one Ter sequence or portions thereof to select against undesired nucleic acid molecules (Fig. 4). Like the positive selection methods described such method may be accomplished using *in vitro* and/or *in vivo* cloning of desired nucleic acid molecules. In one aspect these methods allow selection against undesired starting molecules and/or product molecules during *in vitro* or *in vivo* cloning. For example, the disclosure describes selection against a starting vector molecule which did not receive a desired insert. In another aspect, the disclosure describes selection against intermediates which may be generated during cloning or insertion of nucleic acid molecules. Additionally, the disclosure describes for selection against undesired product molecules generated during cloning reactions.

The disclosure describes assuring a desired orientation of a nucleic acid insert, such as a transposon, into a nucleic acid into which the insert is introduced. By controlling orientation , the whole nucleic acid construct will be allowed to replicate or prevented from replicating. For example, one or more inserts, e.g., transposons, can be contacted with a nucleic acid, e.g., plasmids, BACs, YACs, chromosomes, etc. If one or more of the inserts is in the desired orientation, replication will proceed through the sites that are in the permissive orientation. However, if an insert is oriented such that one or more Ter-sites are in a non-permissive orientation, then replication will not be accomplished. Such methods arc useful whenever an insertion orientation, e.g., the orientation of one or more transposons, is desired and may be especially effective in generating knockout vectors.

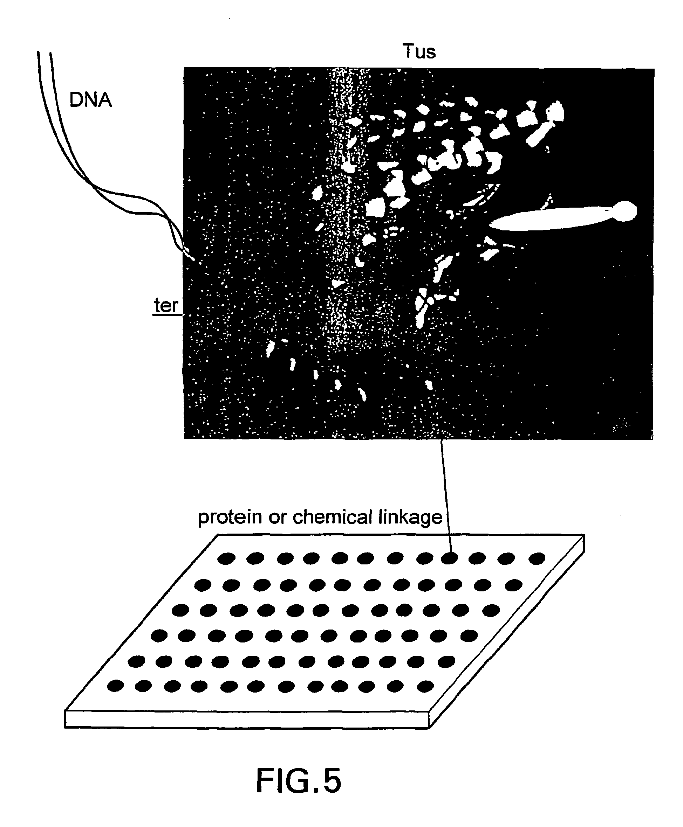

In another aspect, the present invention relates to methods for attaching one or more nucleic acid molecules or populations of nucleic acid molecules to one or more solid supports (Fig. 5). Such methods may comprise binding one or more Ter-binding proteins to one or more solid supports, and contacting the Ter-binding proteins with one or more nucleic acid molecules comprising one or more Ter-sites, wherein the one or more Ter-binding proteins binds to the one or more nucleic acid molecule through interaction at the one or more Ter-sites (or portions thereof). Bound nucleic acid molecules may then be used for further manipulation, for example, by interaction (e.g., hybridization) with one or more oligonucleotides (e.g., primers or probes) or interaction with peptides or proteins. Such manipulations may be more versatile and/or efficient compared to manipulations where other binding methods are used since the invention allows for binding of the nucleic acid molecule of interest to the support at one or more specific sites (depending on the location(s) of the Ter-sites (or portions thereof)). Thus, a nucleic acid of interest may be attached in any orientation with respect to the solid support, i. e., 5', 3', or internal portion proximal to the support. Nucleic acids of the invention may have a double stranded region, a single stranded region and/or a part double stranded part single stranded region on either or both sides of the bound portion of the nucleic acid. In addition, nucleic acids may be attached to a solid support at more than one position of the nucleic acid. This may allow the nucleic acid to be fixed in defined-optionally rigid conformations on a solid support. Non-specific binding methods of the prior art (e.g., nucleic acid molecules at a number of undefined sites such as with the use of poly-lysine coated supports) are unable to accomplish attachment to a solid support in a defined orientation. This aspect of the invention thus may be advantageously used for nucleic acid isolation, for preparing nucleic acid arrays, and for constructing nanodevices.

In another aspect, the present invention relates to a method for attaching a Ter-binding protein and/or fusion protein comprising a Ter-binding site to a solid support, comprising:
directly attaching a nucleic acid molecule comprising one or more Ter-sequences to a solid support; and
contacting the nucleic acid molecule with a Ter-binding protein and/or a fusion protein comprising a Ter-binding site;
wherein the Ter-binding protein and/or fusion protein binds to said nucleic acid molecule through interaction at one or more Ter-sites.
A Ter-binding portion of a fusion protein may be used to, e.g., concentrate, harvest, isolate, etc. a desired component of the fusion protein. Bound Ter-binding proteins and/or fusion proteins may then be further processed. Further processing may comprise, for example, clution and/or cleavage at one or more cleavage sites. In some embodiments, such bound Ter-binding proteins and/or fusion proteins may be interacted with one or more nucleic acid molecules or with other peptides or proteins while still bound to the solid support. In other embodiments, such Ter-binding proteins and/or fusion proteins may be eluted from the solid support prior to further interactions. This aspect of the invention thus may be advantageously used for the isolation or purification of Ter-binding proteins and/or fusion proteins from any sample such as biological samples.

The present disclosure describes a method for improving the transfection efficiency of one or more nucleic acid molecules, comprising providing a Ter-site in the nucleic acid and contacting the nucleic acid with a Ter-binding protein. In some embodiments, the Ter-binding protein may be a modified Ter-binding protein. In some embodiments, the modified protein may comprise one or more receptor binding ligands. In some aspects, the present disclosure describes altered Ter-binding proteins comprising one or more cellular targeting sequences. In some preferred embodiments, one or more of the cellular targeting sequences may be a nuclear localization sequence.

In another aspect, the present disclosure describes methods for enhancing the stability of a linear nucleic acid molecules in vivo, comprising providing a linear nucleic acid molecule, the nucleic acid molecule comprising Ter-sites (or portions thereof) at one or both of its termini, contacting the nucleic acid with a Ter-binding protein to form a stable nucleic acid-protein complex and transfecting the stable nucleic acid-protein complex into a host cell, wherein the complex is more stable and/or more easily transfected than the nucleic acid transfected alone. In some embodiments, the linear nucleic acid comprises a coding sequence.

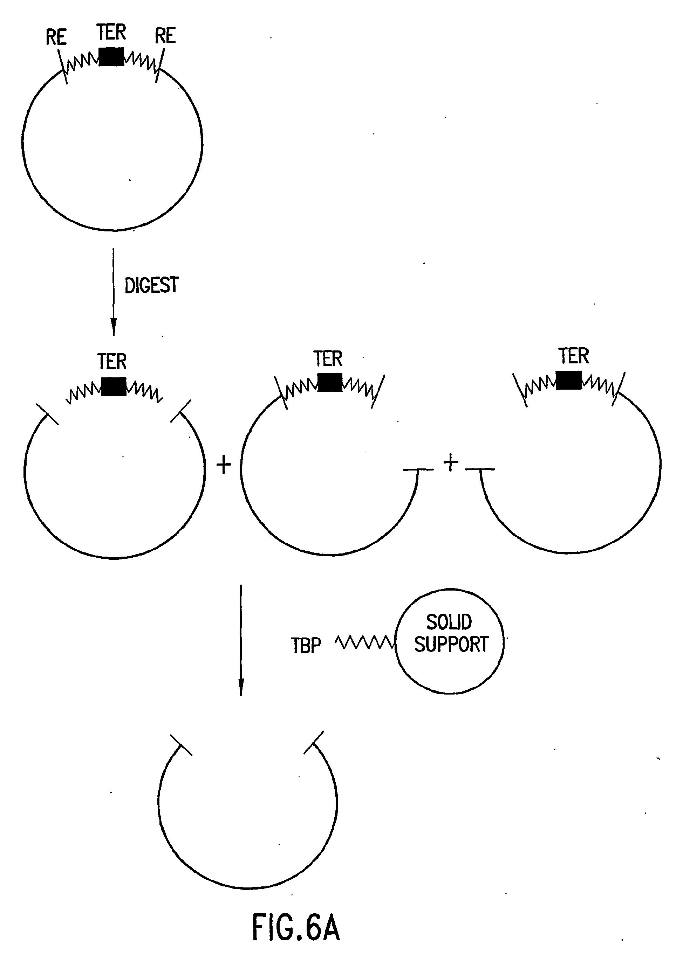

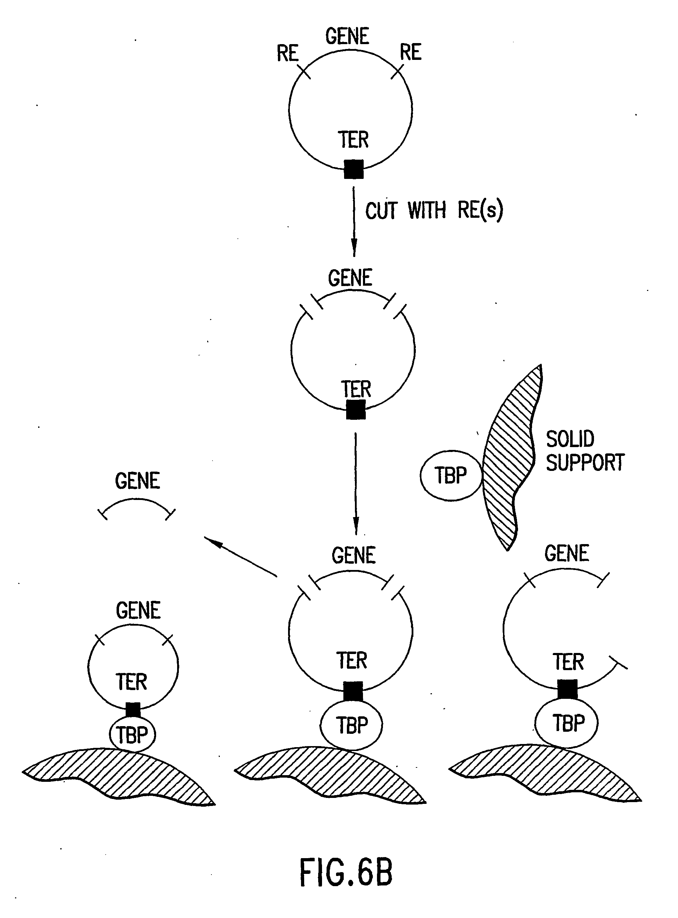

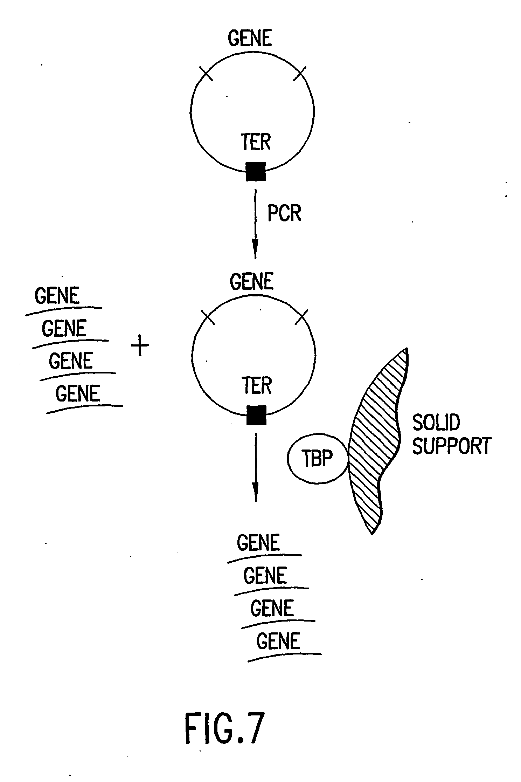

In another aspect, the present disclosure describes a method for isolating a nucleic acid, comprising providing a nucleic acid, the nuclei acid comprising a Ter-site, contacting the nucleic acid with a composition, the composition comprising a Ter-binding protein attached to a solid support, wherein the Ter-binding protein binds to the Ter-site, and isolating or purifying the nucleic acid (Figs. 6A and 6B and Fig. 7). In yet another embodiment, the present disclosure describes improved methods for purification of nucleic acids, especially nucleic acid libraries. Generally, nucleic acids comprising a Ter-site can be separated from other nucleic acids by such methods. One such embodiment is depicted in Figure 6A which shows a stack vector with a stuffer fragment. To prepare vector reagent for library production, the stuffer fragment should be efficiently removed. The present disclosure describes methods for isolating the prepared vector reagent from stuffer fragments. For example, a stock vector can be constructed to comprise a Ter-site in the stuffer fragment. After digestion with restriction enzymes, two cuts with one or more restriction enzyme will result in cleavage of stuffer from prepared reagent. Cuts at only one site or no cuts will leave the stuffer fragment still attached to the vector. Ter-binding protein can be bound to a solid support to effect separation of the stuffer fragments, uncut vectors, and singly cut vectors still comprising stuffer fragment from prepared vector reagent. Ter-binding protein can be bound to any solid support, before, coincident with, or after being reacted with a vector digest. In another embodiment, nucleic acids containing a Ter site, such as uncut plasmids or singly-cut plasmids as well as undesired plasmid materials not containing the desired sequence of interest may thus be removed as shown in Fig. 6B.

In another embodiment, the presence of a Ter site in a template nucleic acid may used as shown in Fig. 7 to remove a template nucleic acid after completion of an amplification reaction, for example, a PCR reaction. The amplified sequence of interest may be the same as that of the template or may be a derivative thereof, e.g., a gene mutated by site directed mutagenesis. In a related aspect, compositions comprising a Ter-binding protein fused to a solid support may comprise for example a slide, a chip, a film, a bead, chromatography media, or a filter.

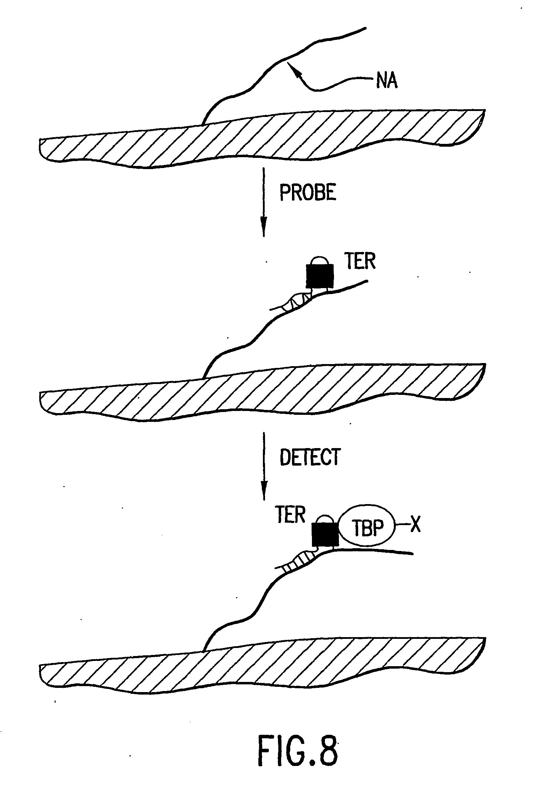

In another aspect, the present disclosure describes methods for detecting a biological molecule, comprising the steps of contacting a biological molecule with reagent, the reagent comprising a nucleic acid portion preferably containing at least one Ter-site and a portion which forms a specific complex with the biological molecule, contacting the complex with a Ter-binding protein optionally comprising a detection molecule, wherein the Ter-binding protein binds to the nucleic acid portions of the reagent, and detecting the bound Ter-binding protein, wherein the presence of the Ter-binding protein correlates to the presence of the biological molecule (Fig. 8). In some embodiments, the detection molecule may be selected from a group consisting of radioisotopes, chromophores, fluorophores, enzymes, antigens, haptens, epitopes and combinations thereof.

In another aspect, a biological molecule can be labeled with a Ter-binding protein. The biological molecule can be, for examples, a polynucleotide, a polypeptide, a polysaccharide, a lipid, or a phospholipid. The biological molecule can then be detected using a polynucleotide comprising a Ter-site which is bound by the Ter-binding protein. This method of detection can be used to amplify a signal for detecting a molecule of interest, for example in an ELISA assay or in a western blot assay.

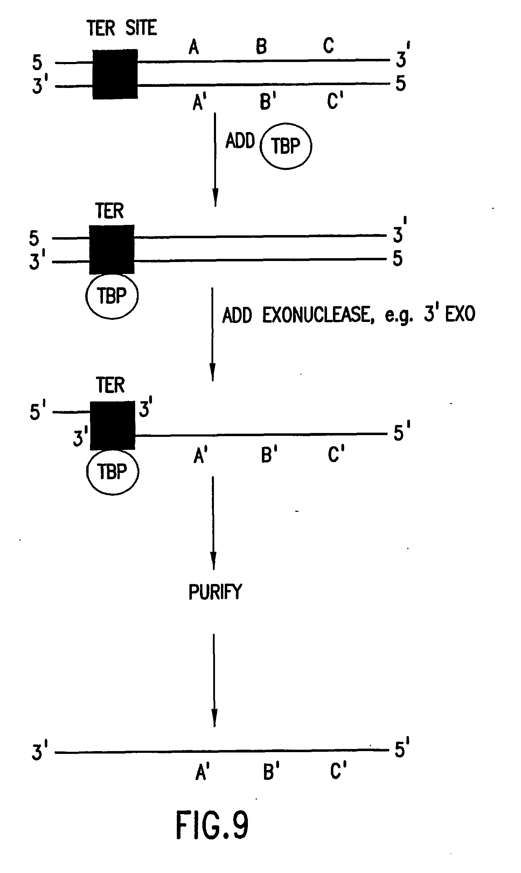

In yet another aspect, the present disclosure describes a method for producing a desired fragment. The method includes binding a Ter-binding protein to the Ter-site on a ds DNA, digesting one strand of DNA with an exonuclease, where the bound Ter-binding protein blocks one strand from digestion with the enzyme. Optionally, the remaining undigested ss DNA may be purified. This can be used to produce a single stranded (ss) DNA fragment from a double-stranded (ds) DNA containing a Ter-site (Fig. 9). Optionally, the ssDNA can be converted to dsDNA or used to produce RNA. RNA yield can be increased by improving initiation efficiency to greater than about 90%, about 95%, in fact approaching 100%.

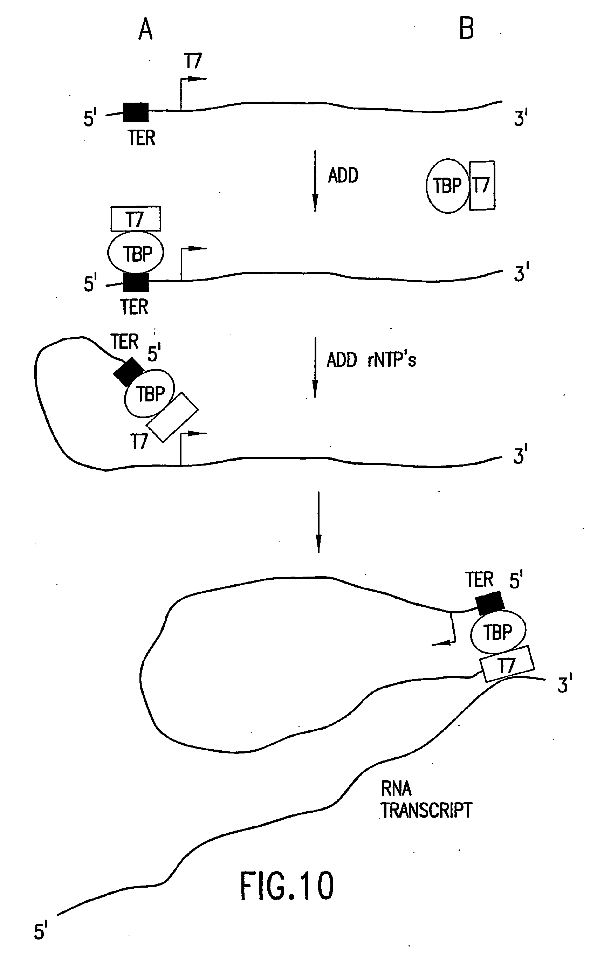

In yet another aspect, the disclosure describes a method for juxtaposing two sites in a nucleic acid molecule, comprising providing a nucleic acid comprising a Ter site in proximity to a promoter, contacting the nucleic acid with a Ter-binding protein that is in functional association with a polymerase, and conducting a polymerization reaction. As shown in Fig. 10, a nucleic acid molecule comprising one or more Ter sites or portions thereof in proximity to one or more promoters may be contacted with a Ter-binding protein to which is attached a functional polymerase enzyme. The one or more Ter-binding sites may be located-such that the polymerase enzyme may functionally engage the promoter and, in the presence of the appropriate cofactors, perform a polymerization reaction. The Ter-binding protein preferably remains bound to the Ter site during the polymerization reaction and the polymerase reaction thus results in pulling the Ter site into proximity with a selected site on the nucleic acid molecule.

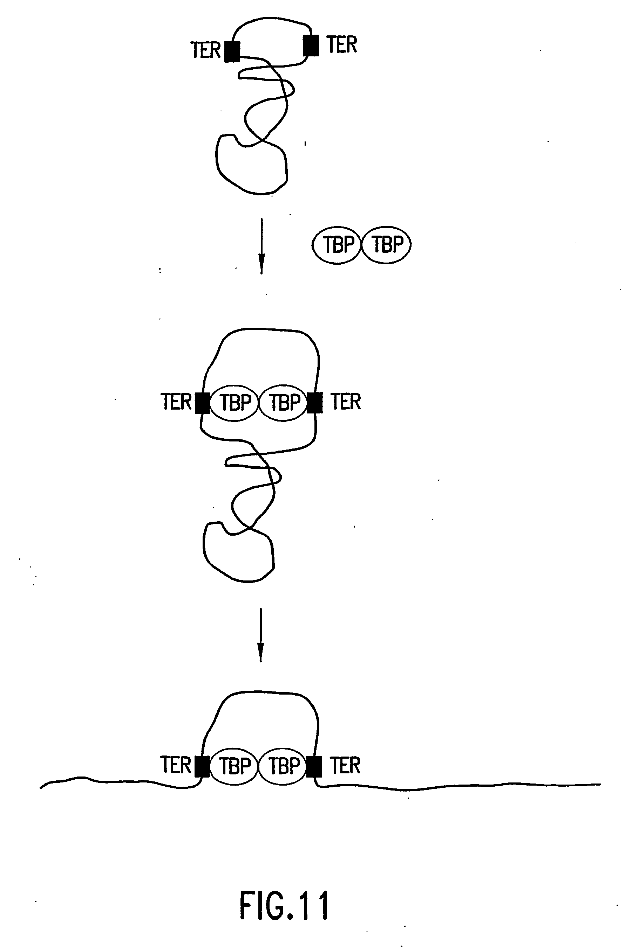

In yet another aspect, the disclosure describes a method for maintaining the topology of a nucleic acid molecule comprising two or more Ter sites. In some aspects, the disclosure describes a method of maintaining the superhelicity of a nucleic acid molecule, comprising contacting a nucleic acid comprising two or more Ter sites with a multivalent Ter-binding protein. In some embodiments, the nucleic acid may be a superc oiled dsDNA containing, e.g., two Ter-sites one at each end of a segment desired to remain supercoilcd after linearization (Fig. 11). A multivalent Ter-binding protein, such as a bivalent Ter-binding protein, is added such that both Ter-sites can be bound and result in isolating one topological domain from another such that one domain can rotate independently of the other. Once the DNA fragment is linearized, the domain bounded by Ter-sites remains in its pre-cleavage topology-supercoiled-until one of the Ter-binding sites is released by the multivalent Tcr-binding protein or until the domain is cleaved. This method is useful for applications where supercoiling is beneficial. In some embodiments, the present disclosure describes a method of supercoiling a linear fragment, comprising contacting a fragment comprising two or more Ter sites with a multivalent Ter-binding protein to form a complex, and contacting the complex with a topoisomerase under conditions in which the topoisomerase supercoils the fragment.

In still another aspect, the present disclosure describes a method for retaining ds DNA duplex under denaturing condition. This can be done by introducing a Tcr-site recognized by a cyclic or thermostable Ter-binding protein into the duplex DNA. Such thermostable Ter-binding protein may be preferably isolated from a thermophilic organism or by cyclizing or otherwise stabilizing a mesophilic Ter-binding protein.

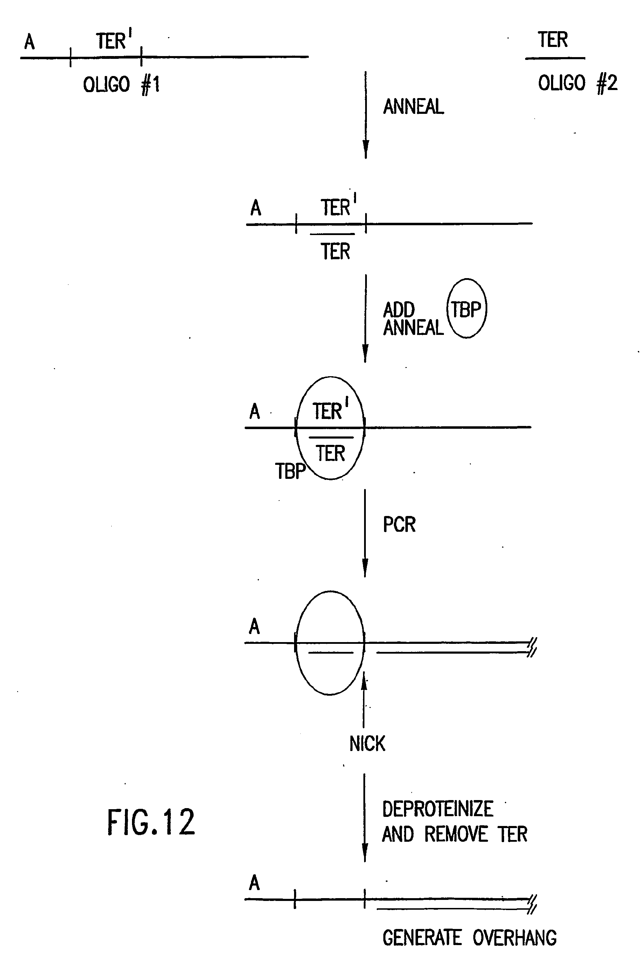

In a similar aspect, the present disclosure describes a method for maintaining a clonal or "sticky end" in a PCR product wherein the primer contains an "overhanging" Ter-site (Fig. 12). Such a ds Ter-site could be distal to the amplified region with respect to the gene specific portion of the primer. The Tcr-site is bound by a Ter-binding protein which is thermostable. Once the PCR reaction is completed and deproteinized, the double stranded DNA product retains a Ter-site overhang.

In another aspect, the present disclosure describes a method for detecting or measuring the proximity of agents to each other. For example, such a method may be used in combination with fluorescence resonance energy transfer (FRET) to measure distances between two molecules of interest. In this method, a Ter-binding protein can be complexed with a molecule which binds the agents to be measured, such as an IgG molecule for example. The complexed Ter-binding proteins can be bound to Ter-sites on nucleic acid molecules of a desired length. The nucleic acid molecules containing the Ter-sites are labeled on the non-Ter-binding end of the molecule. The label can be such that when the two nucleic acid molecules are in close proximity, a change in intensity of label is detected, for example, the label is amplified, or the label is quenched. When the agents are bound by the complexed Ter-binding proteins described above, the distance of the agents can be determined after detecting the signal produced by the label used by knowing the distance occupied by the nucleic acid molecules. This method can be used to detect clustering of receptors of the surface of a cell.

### BRIEF DESCRIPTION OF THE FIGURES

Fig. 1 is a schematic representation of the replication of a plasmid containing a Ter-sites.

Fig. 2 is a schematic representation of the method for using a Ter sequence as a selectable marker. RS = restriction site or recombination site, rep ori = origin of replication arrow indicates direction of replication.

Fig. 3 is a schematic representation of a method for positive selection of a recombinant plasmid using a Ter sequence. GOI = DNA or gene of interest, solid black diamond = 5' end of Ter fragment, solid black circle = 3' end of Ter fragment, rep ori = origin of replication arrow indicates direction of replication.

Fig. 4 is a schematic representation of a method for positive selection for insertion of desired nucleic acid and recombinant plasmids using a Ter sequence. GOI = DNA or gene of interest, solid black diamond = 5' end of Ter fragment, solid black circle = 3' end of Ter fragment, rep ori = origin of replication arrow indicates direction of replication.

Fig. 5 is a schematic representation of the method for attaching nucleic acid to a solid support using a Ter sequence.

. Figs. 6A and 6B are schematic representations of methods for purifying a nucleic acid molecule using the Ter sequence. Fig. 6A shows an embodiment where a Ter site (black box) is present on a stuffer fragment (wavy line) on a plasmid and permits removal of unreacted and partially reacted plasmid using a Ter-binding protein (TBP) attached to a solid support permitting purification of correctly reacted plasmid. Fig. 6B shows an embodiment where a Ter site (black box) is present on a plasmid and permits removal of unreacted and partially reacted plasmid from a reaction mixture reaction using a Ter-binding protein (TBP) attached to a solid support permitting purification of a desired nucleic acid of interest from a reaction mixture. RE = restriction enzyme, TBP=Ter-binding protein.

Fig. 7 is a schematic representation for a method for purifying template containing a Ter-site (black box) from the product of a polymerase chain reaction using a Ter sequence. TBP=Ter-binding protein.

Fig. 8 is a schematic representation of a method for target detection using a Tcr sequence. TBP=Ter-binding protein. X = detection molecule if present.

Fig. 9 is a schematic representation for a method for producing single-stranded nucleic acids using a Ter sequence. TBP=Ter-binding protein.

Fig. 10 is a schematic representation for a method for apposing two ends of the same nucleic acid using a Ter sequence. T7 = T7 RNA polymerase, TBP=Ter-binding protein.

Fig. 11 is a schematic representation for a method for maintaining superhelicity of a region of a linear nucleic acid using a Ter sequence. TBP=Ter-binding protein.

Fig. 12 is a schematic representation for a method for generating overhang "sticky ends" using Ter sequence. A = single stranded exploitable sequence, ter' = bottom strand of duplex Ter sequence, anneal = segment capable of annealing to template, ter = top strand of duplex ter sequence which hybridizes to ter'.

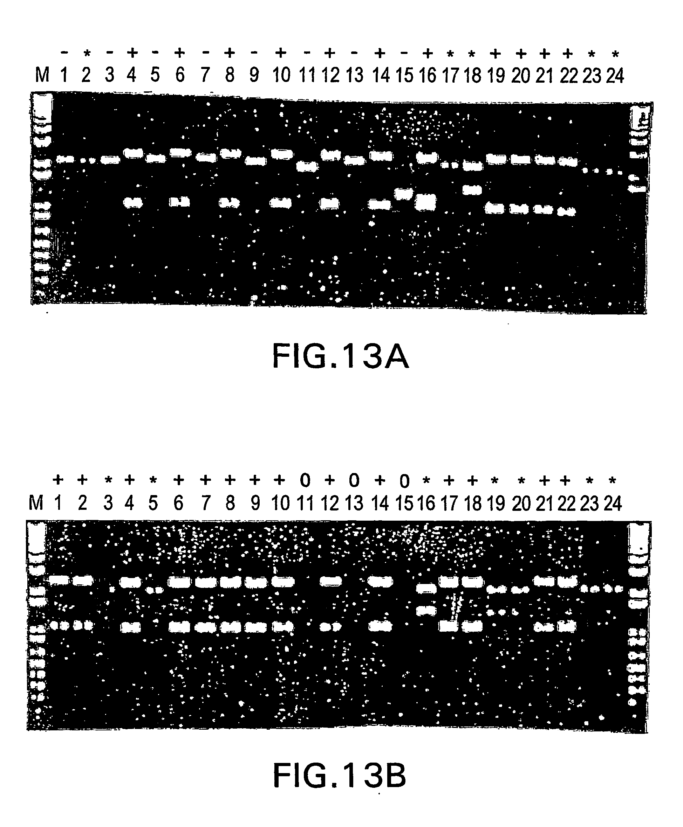

Figs. 13A and 13B demonstrate results of analysis of recombinant vectors using directional cloning with Ter-site. In 13A, the lanes were loaded as follows: M, one kb marker, lanes 1, 3, 5, 7, 9 11,13, and 15, no insert; lanes 2, 4, 6, 8, 10, 12, 14, 16-24, 1µl vector/5 µl insert. In 13B, the lanes were loaded as follows: M one kb marker, lanes 1-24, 10 µl vector/5 µl insert. + = correctly oriented insert, * = backwards insert, - = no insert, 0 = no DNA evident.

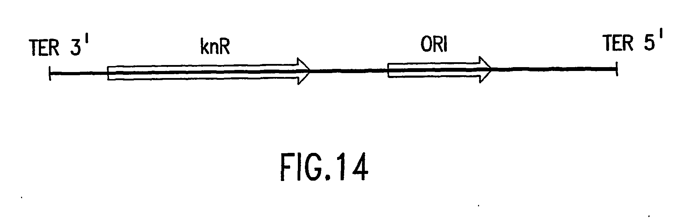

Fig. 14 is a schematic of the construct used in Example 5.

### DETAILED DESCRIPTION OF THE PREFERRED EMBODIMENTS

### Definitions

In the description that follows, a number of terms used in recombinant DNA technology are extensively utilized. In order to provide a clearer and consistent understanding of the specification and claims, including the scope to be given such terms, the following definitions are provided. When a type of molecule is mention, unless contraindicated by the context, the term is seen to include the type of molecule mentioned as well as fragments and derivatives thereof.

Vector A nucleic acid that provides a useful biological or biochemical property to a nucleic acid sequence of interest, for example, an insert, a coding region etc. Examples include plasmids, phages, and other nucleic acid sequences that are able to replicate or be replicated in vitro or in a host cell, or to convey a desired nucleic acid segment to a desired location within a host cell. A vector may comprise various sequences, for example, one or more restriction endonuclease recognition sites and/or recombination sites at which the vector sequences can be manipulated in a determinable fashion without loss of an essential biological function of the vector, and into which a nucleic acid fragment can be inserted, for example, to bring about its replication and/or cloning. Vectors can further provide primer sites, e.g., for PCR, transcriptional and/or transitional initiation and/or regulation sites, recombinational signals, replicons, selectable markers, and other sequences known to those skilled in the art.

Cloning vector. A plasmid, cosmid, viral, or phage DNA or other DNA molecule which is able to replicate autonomously in a host cell, into which DNA may be spliced without loss of an essential biological function of the vector, in order to bring about its replication and cloning. The cloning vector may further contain a marker suitable for use in the identification of cells transformed with the cloning vector. Markers may be, for example, antibiotic resistance genes, e.g., tetracycline resistance or ampicillin resistance.

Expression vector. A vector similar to a cloning vector but which is capable of enhancing the expression of a gene which has been cloned into it, after transformation into a host. The cloned gene is usually placed under the control of (i.e., operably linked to) certain control sequences such as promoter sequences.

Fragment. A fragment is a molecule that may be obtained by cleavage of a larger molecule. Fragments of the present invention may contain at least a portion of a large molecule of the invention. A fragment may be a set of fragments, the set, when properly juxtaposed, forming a complex or a larger molecule. Preferably, the set exhibits one or more functions of the larger molecule.

Recombinnnt host. Any prokaryotic or eukaryotic organism that contains the desired cloned genes in an expression vector, cloning vector or any DNA molecule. The term "recombinant host" is also meant to include those host cells which have been genetically engineered to contain the desired gene on the host chromosome or genome.

Host. Any prokaryotic or eukaryotic organism that is the recipient of a replicable expression vector, cloning vector or any DNA molecule. The DNA molecule may contain, but is not limited to, a structural gene, a promoter and/or an origin of replication.

Promoter. A DNA sequence recognized by an RNA polymerase for specific transcriptional initiation.

Gene. A nucleic acid sequence that contains information necessary for making a biological molecule, such as a polypeptide, protein or RNA. It may include a promoter and/or a structural gene as well as other sequences involved in expression of the molecule.

Polypeptide. As used herein, the term "polypeptide" refers to a sequence of contiguous amino acids, of any length. The terms "peptide," "oligopeptide" or "protein" may be used interchangeably herein with the term "polypeptide."

Derivative. A derivative of a polynucleotide is a molecule having at least 7, 8, or 9 or more preferably at least 10,11, 12,13,14, or 15, or still more preferably 17, 18, 19, 20, 21, 22, 23, 24, or 25 in the same sequence as one or more of the polynucleotides of the invention from which it is derived. One or more of the individual nucleotides of the polynucleotide of the invention may be replaced by one or more insertions, deletions or substitutions to form a derivative. The replacement will preferably not interfere with at least one function of the polynucleotide of the invention. The replacement may be at any position of the polynucleotide, i. e., either end or at an interior location. The replacement may alter one or more characteristics of the polynucleotide, for example, dissociation constant of the polynucleotide from one or more proteins and/or degradation rate-increase or decrease-of the derivative polynucleotide as compared to the polynucleotide from which it is derived. Suitable nucleotide for replacement are known to those of skill in the art and include, but are not limited to, those disclosed below.

A derivative of a polypeptide is a molecule having at least 4, 5, or 6, preferably 7, 8, 9, 10, 11, 12, 13, 14, or 15, more preferably 25, 50, 75, 100. 125, 150, 175, 200, or 250 amino acids in the same sequences as one or more of the polypeptides from which it is derived. One or more of the individual amino acids of the polypeptide may be replaced by one or more insertions, deletions or substitutions to form a derivative. The replacement will preferably not interfere with at least one function of the polypeptide. The replacement may be at any position of the polypeptide, i. c., either end or at an interior location. In some embodiments, all or substantially all of one or more motifs, regions or domains may be deleted. For example, one or more loops -such as the L1 loop of Tus-may be deleted. A derivative may incorporate one or more insertions or substitutions of one or more amino acids-both natural and synthetic amino acids.

A derivative may have the same or different characteristics as the molecule from which it is derived. For example, a derivative polynucleotide may retain the ability to be bound by a wildtype Ter-binding protein. The affinity with which the derivative polynucleotide is bound may be the same as, greater than or lesser than the affinity with which the polynucleotide from which it is derived is bound. A derivative may be a multimer of the molecules-polynucleotides and/or polypeptides. For example, a derivative may be a dimer, trimer, tetramer etc. of the molecules in question. A multimer may be comprised of identical or different monomeric units which may be of the same or different type. For example, a multimer may be of two different polypeptides, two of the same polypeptides of a polypeptide and a polynucleotide.

Operably linked. Operably linked means that a protein or nucleic acid element is positioned so as to influence or be influenced by another protein or nucleic acid element. The elements may be on the same or on different molecules.

Expression. Expression is the process by which a gene produces a polypeptide, protein or RNA. It includes transcription of the gene into an RNA-which may be a messenger RNA (mRNA)-and may include the translation of such mRNA into one or more polypeptides. Those skilled in the art will appreciate that not all RNA molecules are translated into protein, for example ribosomal RNA, and expression in these cases would not include translation.

Substantially Pure. As used herein "substantially pure" means that the desired biomolecule is essentially free from contaminating cellular contaminants that are associated with the desired biomolecule in nature or in a recombinant host in which the biomolecule is produced. Contaminating cellular components may include, but are not limited to, nucleic acids, proteins, lipids and carbohydrates that are not desired.

Primer. As used herein "primer" refers to a single-stranded oligonucleotide that is extended by covalent bonding of nucleotide monomers during amplification or polymerization of a nucleic acid molecule.

Template. The term "template" as used herein refers to a nucleic acid molecule-single strand, double stranded DNA or RNA-that is to be manipulated, for example, amplified, synthesized or sequenced. In the case of a double-stranded nucleic acid molecule, denaturation of its strands to form a first and a second strand may be performed before further manipulations are performed. A primer, complementary to a portion of a template may be hybridized under appropriate conditions and then a nucleic acid polymerase may then synthesize a nucleic acid molecule complementary to all or a portion of the template. The newly synthesized molecule may be longer, equal or shorter in length than the original template. Mismatch incorporation during the synthesis or extension of the newly synthesized nucleic acid molecule may result in one or a number of mismatched base pairs. In addition, the primer used need not be an exact match of the template sequence to which it hybridizes. Mis-matched bases in a primer may be used to effect site directed mutation in a sequence. Thus, the synthesized nucleic acid molecule need not be exactly complementary to the template.

Incorporating. The term "incorporating" as used herein means becoming a part of a nucleic acid molecule or primer.

Amplification. As used herein amplification" refers to any *in vitro* method for increasing the number of copies of a nucleotide sequence with the use of a nucleic acid polymerase, for example, a DNA polymerase, an RNA polymerase and/or a reverse transcriptase. Nucleic acid amplification results in the incorporation of nucleotides into a nucleic acid molecule or primer thereby forming a new nucleic acid molecule complementary to-or substantially complementary to-a nucleic acid template. The newly formed nucleic acid molecule and its template can be used as templates to synthesize additional nucleic acid molecules. As used herein, one amplification reaction may consist of many rounds of nucleic acid replication. DNA amplification reactions include, for example, polymerase chain reactions (PCR). One PCR reaction may consist of, e.g., 5 to 100 "cycles" of denaturation and synthesis of a DNA molecule.

Oligonucleotide. "Oligonucleotide" refers to a synthetic or natural molecule comprising a covalently linked sequence of nucleotides which are joined by a phosphodiester bond between the 3' position of the pentose of one nucleotide and the 5' position of the pentose of the adjacent nucleotide.

Nucleotide. As used herein "nucleotide" refers to a base-sugar-phosphate combination. Nucleotides are monomeric units of a nucleic acid sequence (DNA and RNA). The term nucleotide includes deoxyribonucleoside triphosphates such as dATP, dCTP, dITP, dUTP, dGTP, dTTP, or derivatives thereof. Such derivatives include, for example, [α-S]dATP, 7-deaza-dGTP and 7-deaza-dATP. The term nucleotide as used herein also refers to dideoxyribonucleoside triphosphates (ddNTPs) and their derivatives. Illustrative examples of dideoxyribonucleoside triphosphates include, but are not limited to, ddATP, ddCTP, ddGTP, ddITP, and ddTTP. According to the present invention, a "nucleotide" may be unlabeled or detectably labeled by well known techniques. Detectable labels include, for example, radioactive isotopes, fluorescent labels, chemiluminesecnt labels, bioluminescent labels and enzyme labels.

Thermostable. As used herein "thermostable" refers to a Ter-binding protein that is resistant to inactivation by heat. Ter-binding proteins bind a Ter-site on a nucleic acid molecule. For mesophilic Ter-binding proteins, the binding can be reduced-transiently or permanently—by heat treatment. As used herein, a thermostable Ter-binding activity is more resistant to heat inacti vation than a mesophilic Ter-binding protein. However, a thermostable Ter-binding protein does not mean to refer to a protein that is totally resistant to heat inactivation and thus heat treatment may reduce the Ter-binding activity to some extent.

Hybridization. The terms "hybridization" and "hybridizing" refers to the pairing of two complementary single-stranded nucleic acid molecules (RNA and/or DNA) to give a double-stranded molecule. As used herein, two nucleic acid molecules may be hybridized, although the base pairing is not completely complementary. Accordingly, mismatched bases do not prevent hybridization of two nucleic acid molecules provided that appropriate conditions, well known in the art, are used.

Ligation, The covalent attachment between a first and a second nucleotide sequence.

Target polynucleotide sequence. All or a portion of a sequence of nucleotides to be identified, the identity of which is known to a sufficient extent so as to allow the preparation of a binding polynucleotide sequence that is complementary to and will hybridize with such target polynucleotide sequence. The target polynucleotide sequence usually will contain from about 12 to 1000 or more nucleotides, preferably 15 to 50 nucleotides. The target polynucleotide sequence may or may not be a portion of a larger molecule.

Termination sequence, A termination sequence is a nucleic acid molecule comprising a sequence of nucleotides that can be recognized-i.e., bound-by one or more Ter-binding protein or peptides and/or replication termination proteins or peptides.

### Ter-sites.

Ter-sites according to the invention are any replication termination sequence from any source including those found in eukaryotic and prokaryotic (including gram positive and gram negative microorganisms). The invention also contemplates any portion of such Ter-sites that may be recognized and bound by one or more Ter-binding proteins such as replication terminator proteins or peptides. A portion of a Ter-site may comprise from about 6, 7, 8 or more nucleotides of a Ter-site but less than an entire site. In some aspects, a Ter-site may comprise a double-stranded nucleic acid composition, e.g., a double-stranded molecule one strand of which comprises a sequence listed in Table 1 and the other strand having a sequence complementary to the first strand, or a single stranded nucleic acid comprising a sequence from Table 1 or a single stranded molecule comprising a sequence complementary to a sequence in Table 1. The invention is also directed to mutant or derivative Ter-sites (and portions and combinations thereof) that have increased or decreased ability to he bound by such Ter-binding proteins or peptides. Mutant or derivative Ter-sites for use in the invention may be made by standard mutagenesis techniques (to make deletions, substitutions and insertions in the sequence of interest) or desired derivative Ter-sitcs may be made by standard chemical synthesis techniques (e.g., oligonucleotide synthesis). Ter-sites for use in the invention have been identified in a variety of organisms and plasmids. Table 1 presents the nucleotide sequences of a representative number of sites from *E. coli* and related species as well as plasmids and a number of *Bacillus* species.

**Table 1**

| *E. coli* | |
|---|---|
| TerA | AATTA GTATG TTGTA ACTAA AGT (SEQ ID NO:1) |
| TcrB | AATAA GTATG TTGTA ACTAA AGT (SEQ ID NO:2) |
| TerC | ATATA GGATG TTGTA ATTAA TAT (SEQ ID NO:3) |
| TerD | CATTA GTATG TTGTA ACTAA ATG (SEQ ID NO:4) |
| TerE | TTAAA GTATG TTGTA ACTAA G (SEQ ID NO:5) |
| TerF | CCTTC GTATG TTGTA ACGAC GAT (SEQ ID NO:6) |
| TerG | GATGA GTATG TTGTA ACTAA CTA (SEQ ID NO:7) |
| | |

| *S. typhimurium* | |
|---|---|
| TerA | ATTAA GTATG TTGTA AATAA AGC (SEQ ID NO:8) |
| Ter(amyA) | GATGA GTATG TTGTA ACTAA ATG (SEQ ID NO:9) |
| | |

| Plasmids | |
|---|---|
| R6KterR1 | CTCTT GTGTG TTGTA ACTAA ATC (SEQ ID NO:10) |
| R6KterR2 | CTATT GAGTG TTGTA ACTAC TAG (SEQ ID NO:11) |
| R100TerR1 | ATTAT GAATG TTGTA ACTAC TTC (SEQ ID NO: 12) |
| R100TerR2 | TGTCT GAGTG TTGTA ACTAC AGC (SEQ ID NO:13) |
| RITerR1 | ATTAT GAATG TTGTA ACGAC ATC (SEQ ID NO:14) |
| RITerR2 | TTTTT GTGTG TTGTA ACTAA ATT (SEQ ID NO:15) |
| RepFICferR1 | ATTAT GAATG TTGTA ACTAC ATT (SEQ ID NO:16) |
| St90kbTer | ATTTT GGATG TTGTA ACTAT TTG (SEQ ID NO:17) |
| | |

| *Bacillus spp.* | |
|---|---|
| *B*. *atrophaeus* | |
| TerI GAACT | AAATA AACTA TGTAC CAAATGTTCA (SEQ ID NO:18) |
| TerII TAACT | GAAAA CACTA TGTAC TAAAT ATTCA (SEQ ID NO:19) |
| | |

| *B. mojavensis* | |
|---|---|
| TerI | GAACA AAAAA AACTA TGTAC CAAAT GTTCA (SEQ ID NO:20) |
| TerII | AAACT GAGAA TACTA TGTAC TAAAT ATTCA (SEQ ID NO:21) |
| | |

| *B. vallismortis* | |
|---|---|
| TerII | ATACT AAAAA TATGA TGTAC TAAAT ATTCA (SEQ ID NO:22) |

| *B. amyloliquefaciens* | |
|---|---|
| TerII | TAACA AATTA TTCCA TGTAC TAAAT ATTCT (SEQ ID NO:23) |
| | |

| *B. subtilis* 168 | |
|---|---|
| TerVIII | GAACT AATTA AACTA TGTAC TAAAT TTTCA (SEQ ID NO: 24) |
| TerIX | ATACT AATTG ATCCA TGTAC TAAAT TTTCA (SEQ ID NO:25) |

The nucleotide sequences of the various Ter-sites presented in Table 1 indicate that certain positions are highly conserved. In *E. coli* the G at residue 6 and the 11 bases starting with position 8 and ending with position 19 are conserved in all Ter-sites with the sole exception of a T/G modification at position 18 of the TerF sequence. In *Bacillus* nuclcotidcs 3-5, 7, 13, 15, 16-20, and 22-25 of the sequences in Table 1 are highly conserved.

The present invention contemplates the use of Ter-sites and Ter-binding proteins from any source. In some embodiments, the Ter-sites and Ter-binding proteins may be derived from prokaryotes, for example, thermophilic organisms such as, for example, *B. stearothermophilus.* Other sources include *Enterobacteriaceae* or eukaryotes.

Ter-sites that have been altered by removing a portion of the sequence or by substitution or mutation and that still (1) retain the ability to bind Ter-binding protein are included as part of this invention and/or (2) still retain directionality are included as part of this invention. Functional domains and regions of Ter sites necessary for proper function are described in Coskun-Ari and Hill, J. Biol. Chem. 17 272:26448-26456 (1997). Ter-sites that are altered such that a Ter-binding protein binds with less affinity are also useful in reactions where, for example, manipulation of replication termination is desired (Coskun-Ari and Hill, 1997; Sharma and Hill, Mol. Microbiol. 18:45-61 (1995)).

Nucleic acids comprising the Ter sites of the invention may be prepared using any convention technology, for example, chemical synthesis using phosporamidite chemistry or amplification techniques, i.e., PCR and the like. Optionally, detectable molecules may be attached to the nucleic acids comprising the Ter sites. Suitable detection molecules are known to those skilled in the art and include, but are not limited to, enzymes such as horseradish peroxidase, alkaline phosphatase, luciferase, beta-galactosidase and beta-glucuronidase, fluorescent moieties, chromophores, haptens and/or epitopes recognized by an antibody. Detection molecules may be attached during synthesis, for example, by using chemically modified nucleotides-for example, fluorescently labeled-during an amplification reaction. In some instances it may be desirable to introduce a detection molecule after synthesis of the nucleic acid, for example, by chemically coupling the detection molecule to the nucleic acid.

Oligonucleotides comprising Ter-sites may be single or double stranded. In some embodiments, oligonucleotides may be in the form of a hairpin or stem-loop such that one portion of the oligonucleotide hybridizes to another portion of the oligonucleotide to form a double stranded portion of the oligonucleotide comprising all or a portion of a Ter-site.

### Ter-binding proteins.

One example of a Ter-binding protein is a replication terminator protein (RTP). An RTP is a sequence specific DNA-binding protein which, when bound to the double stranded termination sequence, allows replication arrest. The RTP from *E*. *coli* is a 36,000 Da protein designated Tus (also tau). The Tus protein binds Ter-sites as a monomer. Tus binds the TerB site extremely tightly with a dissociation constant of up to 3 X 10⁻¹³ M *in vitro* (depending on the buffer conditions). The binding of Tus to other Ter-sites is somewhat less tight with dissociation constants on the order of 10⁻¹⁰ to 10⁻¹¹ M. Preferred Ter-binding proteins of the present invention may have a dissociation constant from a Ter-site of from about 10⁻⁹ M to about 10⁻¹⁵ M, from about 10⁻¹⁰ M to about 10⁻¹⁴ M, or from about 10⁻¹¹ M to about 10⁻¹³ M.

The Tus-TerB complex is very stable with a half-life of up to 550 minutes. The DNA sequence of the Tus gene is known (sec, Hidaka, M., et al., Purification of a DNA replication terminus (ter) site-binding protein in Escherichia coli and identification of the structural gene, J. Biol. Chem. 264 (35):21031-21037 (1989) and Hill, T.M., et al., Tus, the trans-acting gene required for termination of DNA replication in Escherichia coli, encodes a DNA-binding protein, Proc. Natl. Acad. Sci. U.S.A. 86 (5):1593-1597 (1989)). Strains of *E*. *coli* that lack functional Tus protein are known. The crystal structure of the protein in a complex with a Ter-site has been produced (Bussiere, et al., Molecular Microbiology 31 (6): 1611-1618 (1999)).

Mutants and variants of Ter-binding proteins still able to bind or with altered ability to bind for use in certain applications are part of the present invention. Such mutants include those with mutations in the DNA-binding domain such as, E49, H50, K89, T136, K175, I177, R198, R232, V234, K235, Q237, Q252, A254, R288, K290 (Skokotas et al., J. Biol. Chem. 270:30941-30948 (1995)), among others. Functional domains of some Ter-binding proteins have been defined and may be altered to increase or decrease its ability to bind Ter, for example, mutants in the replication fork blocking domain include, H31, K32, L33, L34, V35, A36, R37, I62, V97, L98, C99, Y100, Q101, V102, D103, N104, S106, Q107, L110, V161, L162, H136, D164, P165, A166, T167, L168, R169, P170, R241, V242, W243, Y244, K245, G246, D247, Q248, L259, I260, A261, L262, N264, R265, D266, N267, G268, A269, G270, V271, P272, D273, V274, G275 (Duggin et al, J. mol. Biol. 286:1325-1335 (1999)) among others. For example, a cyclized Ter-binding protein which is resistant to denaturation by chemicals may be used to prevent duplex DNA from denaturing during such conditions. The cyclized protein can further be labeled to detect double stranded nucleic acid.

Also included are Ter-binding proteins that are derived from thermostable organisms as well as those derived from hypothermophiles or psychrophiles.

The present invention also comprises modified Ter-binding proteins. The modified Ter-binding protein may be a full length Ter-binding protein or a portion of a Ter-binding protein that retains the ability to bind a Ter site. The modifying moieties may he covalently attached to the Ter-binding protein, for example, by coupling using those coupling reagents known to those skilled in the art. Suitable coupling reagents are commercially available from, for examples, Pierce Chemical Co., Rockford, IL.

In some embodiments, the modifying moiety may be a polypeptide and the peptide backbone of the polypeptide may be contiguous with the peptide backbone of the Ter-binding protein forming a fusion protein between the Ter-binding protein and the modifying polypeptide. The construction of fusion proteins is routine in the art. Any suitable polypeptide may be fused to all or a portion of a Ter-binding protein. The polypeptide may be fused at the N-terminal of the Ter-binding protein, the C-terminal of the Ter-binding protein or at an interior position of the Ter-binding protein. Any site of fusion may be used so long as the binding capability of the Ter-binding protein is not substantially reduced. In this context, substantially reduced indicates that the modified Ter-binding protein does not bind a Ter site with sufficient affinity to allow detection of the modified Ter-binding protein.

Any desired modifying group may be attached to a Ter-binding protein for use in the present invention by chemical coupling and/or by preparation of a fusion protein. In some embodiments, the modifying group may be a ligand for a receptor. Ligands for use in the present invention may be ligands for cell surface receptors including, but not limited to, the transferrin receptor, the serum albumin receptor, the asialoglycoprotein receptor, an adenovirus receptor, a retrovirus receptor, CD4, lipoprotein (a) receptor, immunoglobulin Fc receptor, α-fetoprotein receptor, LDLR-like protein (LRP) receptor, acetylated LDL receptor, mannose receptor, or mannose-6-phosphate receptor. Many other cell surface receptors and their associated ligands are known to those skilled in the art and modified Ter-binding proteins comprising these ligands are within the scope of the present invention. For a detailed list of receptors and ligands and their use to transport molecules into cells see United States Patent 6,331,289, issued to Klaveness, et al., and United States Patent 6,262,026, issued to Heartlein, et al. A modified Ter-binding protein comprising a ligand for a cell surface receptor can be used as a means by which nucleic acids comprising a Ter site can be transported into cells. Proteins comprising a Ter-binding protein and a ligand for one or more receptors may be contacted with a nucleic acid comprising a Ter site in order to form a complex of nucleic acid-Ter-binding protein-ligand. The complex may then be brought into contact with a cell expressing the appropriate receptor resulting in the up take of the complex into the target cell. Suitable receptor are present on a wide variety of different cell types and allow uptake of nucleic acids comprising a Ter site into a wide variety of cell types.

In some embodiments, a Ter-binding protein may comprise a detection molecule. Suitable detection molecules are known to those skilled in the art and include, but are not limited to, enzymes with detectable activities such as horse radish peroxidase, alkaline phosphatase, luciferase, beta-galactosidase and beta-glucuronidase, fluorescent moieties, chromophores, haptens and/or epitopes recognized by an antibody. In some preferred embodiments, the detection molecule may comprise combinations of fluorescent moieties, chromophores, enzymes, haptens and/or epitopes and the like. Detection molecules may be covalently attached to a Ter-binding protein by chemical coupling and/or by construction of a fusion protein.

In some embodiments, the modified Ter-binding proteins may comprise a cellular targeting sequence. Such a sequence directs the Ter-binding protein and any nucleic acid bound by the protein to one or more specific locations in an organism or cell. In some embodiments, the cellular targeting sequence may be a nuclear localization sequence and the Ter-binding protein and bound nucleic acid may be directed to the nucleus of a target cell. Cellular targeting sequences may also help reduce or prevent degradation of the nucleic acid molecule, for example, degradation occuring in the endosomes and/or lysomes. Suitable cellular targeting sequences are known to those skilled in the art and may be derived from any source, for example, from viral proteins. For examples of suitable cellular targeting sequences as well as examples of suitable ligands and other polypeptide portions that may be used to modify the Ter-binding proteins see United States Patent 6,177,554, issued to Woo, et al.

In some embodiments, the present invention provides a fusion protein comprising a Ter-binding protein and a polypeptide or protein of interest. The presence of the Ter-binding protein permits the detection and/or affinity purification of the polypeptide or protein of interest using an oligonucleotide comprising a Ter site. For example, an oligonucleotide comprising a Ter site may be attached to a solid support, for example, a bead, a chromatography support and the like. The fusion protein comprising a Ter-binding portion and a polypeptide of interest may then be contacted with the solid support under conditions—pH, ionic strength, temperature and the like—that permit the binding of the Ter-binding portion of the fusion protein to the oligonucleotide. Any contaminating molecules may be washed from the solid support and the bound fusion protein may be eluted. The fusion proteins may optionally comprise one or more cleavage sites for proteolytic enzymes. In some embodiments, one or more cleavage sites may be located between the Ter-binding portion of the fusion protein and one or more additional polypeptide portions. The construction of fusion proteins comprising cleavage sites is well known in the art, see, for example, Riggs, et al., in Current Protocols in Molecular Biology, Ausubel, et al. Eds., John Wiley & Sons, Inc. Chapter 16, pages 16.4.1-16.4.4, 1997.

In some embodiments, the modified Ter-binding proteins may comprise more than one Ter-binding portions. When two or more Ter-binding portions are linked, they may be from the same or different Ter-binding proteins and have the same or different affinities for Ter sites. Multiple Ter-binding proteins may be linked by chemically coupling Ter-binding proteins or by the creation of fusion proteins. The multivalent Ter-binding proteins can be made by cloning—with or without linkers—direct repeats of the open reading frame encoding a Ter-binding protein or by crosslinking the two molecules, for example. Modified Ter-binding proteins comprising multiple Ter-binding portions may also further comprise additional modifications, for example, detection molecules, ligands and other modifications.

In some embodiments, a Ter-binding protein may comprise more than one modification. For example, a Ter-hinding protein may comprise a ligand for a cell surface receptor and a detection molecule. A configuration of this sort will allow detection of the uptake of the modified Ter-binding protein, preferably provide the ability to detect a complex of the modified Ter-binding protein and a nucleic acid to which it is bound.

### Solid supports and arrays.

Supports for use in accordance with the invention may be any support or matrix suitable for attaching nucleic acid molecules comprising one or more Ter sites or portions thereof and/or molecules comprising all or a portion of a Ter-binding protein. Such molecules may be added or bound (covalently or non-covalently) to the supports of the invention by any technique or any combination of techniques well known in the art. Supports of the invention may comprise silicon, biochips, nitrocellulose, diazocellulose, glass, polystyrene (including microtitre plates), polyvinylchloride, polypropylene, polyethylene, polyvinylidenedifluoride (PVDF), dextran, Sepharose, agar, starch and nylon. Supports of the invention may be in any form or configuration including beads, filters, membranes, sheets, frits, plugs, columns and the like. Solid supports may also include multi-well tubes (such as microtitre plates) such as 12-well plates, 24-well plates, 48-well plates, 96-well plates, and 384-well plates. Preferred beads are made of glass, latex or a magnetic material (magnetic, paramagnetic or superparamagnetic beads).

Attachment of molecules to solid supports is well known in the art. For example, U.S. Pat. No. 5,384,261 is directed to a method and device for forming large arrays of polymers on a substrate. According to a preferred aspect of the invention, the substrate is contacted by a channel block having channels therein. Selected reagents are flowed through the channels, the substrate is rotated by a rotting stage, and the process is repeated to form arrays of polymers on the substrate. The method may be combined with light-directed methodologies.

U.S. Patent 5,744,305 is another exemplary teaching showing for example, that selectively removable protecting groups allow creation of well defined areas of substrate surface having differing reactivities. The protecting groups can be selectively removed from the surface by applying a specific activator, such as electromagnetic radiation of a specific wavelength and intensity. The specific activator can expose selected areas of surface to remove the protecting groups in the exposed areas.

Protecting groups are used in conjunction with solid phase oligomer syntheses, such as peptide syntheses using natural or unnatural amino acids, nucleotide syntheses using deoxyribonucleic and ribonucleic acids, oligosaccharide syntheses, and the like. In addition to protecting the substrate surface from unwanted reaction, the protecting groups block a reactive end of the monomer to prevent self-polymerization. For instance, attachment of a protecting group to the amino terminus of an activated amino acid, such as an N-hydroxysuccinimide-activated ester of the amino acid, prevents the amino terminus of one monomer from reacting with the activated ester portion of another during peptide synthesis. Alternatively, a protecting group may be attached to the carboxyl group of an amino acid to prevent reaction at this site. Most protecting groups can be attached to either the amino or the carboxyl group of an amino acid, and the nature of the chemical synthesis will dictate which reactive group will require a protecting group. Analogously, attachment of a protecting group to the 5'-hydroxyl group of a nucleoside during synthesis using for example, phosphate-triester coupling chemistry, prevents the 5'-hydroxyl of one nucleoside from reacting with the 3'-activated phosphate-triester of another.

Regardless of specific use, protecting groups are employed to protect a moiety on a molecule from reacting with another reagent. Protecting groups of the present invention have the following characteristics: they prevent selected reagents from modifying the group to which they arc attached; they are stable (that is, they remain attached to the molecule) to the synthesis reaction conditions; they are removable under conditions that do not adversely affect the remaining structure; and once removed, do not react appreciably with the surface or surface-bound oligomer. The selection of a suitable protecting group will depend, of course, on the chemical nature of the monomer unit and oligomer, as well as the specific reagents they are to protect against.

Protecting groups are sometimes photoactivatable. The properties and uses of photoreactive protecting compounds have been reviewed. See, McCray et al., Ann. Rev. of Biophys. and Biophys. Chem. (1989) 18:239-270. Photosensitive protecting groups can be removable by radiation in the ultraviolet (UV) or visible portion of the electromagnetic spectrum. Protecting groups can be removable by radiation in the near UV or visible portion of the spectrum. Activation may also be performed by other methods such as localized heating, electron beam lithography, laser pumping, oxidation or reduction with microelectrodes, and the like. Sulfonyl compounds are suitable reactive groups for electron beam lithography. Oxidative or reductive removal is accomplished by exposure of the protecting group to an electric current source, preferably using microelectrode directed to the predefined regions of the surface which are desired for activation. Other methods may be used in light of this disclosure. Many, although not all, of the photoremovable protecting groups will be aromatic compounds that absorb near-UV and visible radiation. Suitable photoremovable protecting groups are described in, for example, McCray et al., Patchornik, J. Amer. Chem. Soc. (1970) 92 :6333, and Amit et al., J. Org. Chem. (1974) 39:192.

In a preferred aspect, methods of the invention may be used to prepare arrays of proteins and/or nucleic acid molecules (RNA or DNA) or arrays of other molecules, compounds, and/or substances. Such arrays may be formed on microplates, glass slides or standard blotting membranes and may be referred to as microarrays or gene-chips depending on the format and design of the array. Uses for such arrays include gene discovery, gene expression profiling, genotyping (SNP analysis, pharmacogenomics, toxicogenetics), and the preparation of nanotechnology devices.

Synthesis and use of nucleic acid arrays and generally attachment of nucleic acids to supports have been described (*see, e.g.,* U.S. Patent No. 5,436,327, U.S. Patent No. 5,800,992, U.S. Patent No. 5,445,934, U.S. Patent No. 5,763,170, U.S. Patent No. 5,599,695 and U.S. Patent No. 5,837,832). An automated process for attaching various reagents to positionally-defined sites on a substrate is provided in Pirrung, et al. U.S. Patent No. 5,143,854 and Barrett, et al. U. S. Patent No. 5,252,743. For example, disulfide-modified oligonucleotides can be covalently attached to solid supports using disulfide bonds. (*See* Rogers et al., Anal. Biochem. 266:23-30 (1999).) Further, disulfide-modified oligonucleotides can he peptide nucleic acid (PNA) using solid-phase synthesis. (*See* Aldrian-Herrada et al., J. Pept. Sci. 4:266-281 (1998).) Thus, nucleic acid molecules comprising one or more Ter sites or portions thereof can be added to one or more supports (or can be added in arrays on such supports).

The attachment of polypeptides to solid supports is well known in the art. For example, Deutsch, et al., U.S. Patent No. 4,615,985, describe the attachment of proteins to a nylon support, Ikeda, et al., U.S. Patent No. 4,582,622, describe the attachment of proteins to magnetic particles, Burton, et al., U.S. Patent No. 5,998,155, describe the attachment of biotin binding proteins to solid supports, and Wagner, U.S. Patent No. 6,120,992, describes the attachment of nucleic acid binding proteins to solid supports and their subsequent use to bind nucleic acids. The Ter-binding proteins of the present invention may be attached to a solid support and subsequently used to bind nucleic acid molecules comprising a Ter site.

Essentially, any conceivable support may be employed in the invention. The support may be biological, non-biological, organic, inorganic, or a combination of any of these, existing as particles, strands, precipitates, gels, sheets, tubing, spheres, containers, capillaries, pads, slices, films, plates, slides, etc. The support may have any convenient shape, such as a disc, square, sphere, circle, etc. The support is preferably flat but may take on a variety of alternative surface configurations. For example, the support may contain raised or depressed regions which may be used for synthesis or other reactions. The support and its surface preferably form a rigid support on which to carry out the reactions described herein. The support and its surface are also chosen to provide appropriate light-absorbing characteristics. For instance, the support may be a polymerized Langmuir Blodgett film, functionalized glass, Si, Ge, GaAs, GaP, SiO₂, SIN₄, modified silicon, or any one of a wide variety of gels or polymers such as (poly)tetrafluoroethylene, (poly)vinylidenedifluoride, polystyrene, polycarbonate, or combinations thereof. Other Support materials will be readily apparent to those of skill in the art upon review of this disclosure. In a preferred embodiment the support is flat glass or single-crystal silicon.

Thus, the invention provides methods for preparing arrays of nucleic acid molecules directly attached to supports. In some embodiments, these nucleic acid molecules will have one or more Ter sites at one or more (*e.g*., one, two, three or four) positions in the nucleic acid molecule. In some additional embodiments, one nucleic acid molecule may be attached directly to the support, or to a specific section of the support, and one or more additional nucleic acid molecules will be indirectly attached to the support via attachment to the nucleic acid molecule which is attached directly to the support. In such cases, the nucleic acid molecule which is attached directly to the support provides a site of nucleation around which a nucleic acid array may be constructed.

In one aspect, the invention provides supports containing nucleic acid molecules containing Ter sites (directly attached to the supports). In some embodiments, the nucleic acid molecules of these supports will contain at least one Ter site. These bound nucleic acid molecules are useful, for example, for identifying other nucleic acid molecules (*e.g*., nucleic acid molecules which hybridize to the bound nucleic acid molecules under stringent hybridization conditions) and proteins which have binding affinity for the bound nucleic acid molecules. The Ter sites may be composed of two separate oligonucleotides or may be a single nucleotide in a stem-loop or hairpin configuration. Stem-loop and hairpin oligonucleotides may form a functional Ter site under conditions that permit the hybridization of complementary regions of the oligonucleotide that comprise all or a portion of a Ter site. This will be particularly useful to for the reversible binding of Ter-binding protein containing molecules. The Ter-binding protein containing molecule may he bound to the double stranded portion of the stem-loop or hairpin oligonucleotide comprising all or a portion of the Ter site and then may be eluted from the oligonucleotide by changing the conditions—pH, salt ionic strength, temperature etc.—such that the hybridized portion of the oligonucleotide becomes all or partially single stranded such that the Ter-binding protein no longer binds to the Ter site.

In some embodiments, expression products may also be produced from these bound nucleic acid molecules while the nucleic acid molecules remain bound to the support. Thus, compositions and methods of the invention can be used to identify expression products and products produced by these expression products.

Further, nucleic acid molecules attached to supports may be released from these supports. Methods for releasing nucleic acid molecules include restriction digestion, recombination, and altering conditions (*e.g*., temperature, salt concentrations, etc.) to induce the dissociation of nucleic acid molecules which have hybridized to bound nucleic acid molecules. Thus, methods of the invention include the use of supports to which nucleic acid molecules have been bound for the isolation of nucleic acid molecules.

Examples of compositions which can be formed by binding nucleic acid molecules to supports are "gene chips," often referred to in the art as "DNA microarrays" or "genome chips" (*see* U.S. Patent Nos. 5,412,087 and 5,889,165, and PCT Publication Nos. WO 97/02357, WO 97/43450, WO 98/20967, WO99/05574, WO99/05591, and WO99/40105. In various embodiments of the invention, these gene chips may contain two- and three-dimensional nucleic acid arrays described herein.

The addressability of nucleic acid arrays of the invention means that molecules or compounds which bind to particular nucleotide sequences can be attached to the arrays. Thus, components such as proteins and other nucleic acids can be attached to specific locations/positions in nucleic acid arrays of the invention.

### Selection Methods

Incorporation of all or a portion of a Ter site into a vector and/or a nucleic acid of interest may permit the selection of desired nucleic acids that either do not contain a Ter site (negative selection) or do contain a sequence of interest (positive selection). With reference to Fig. 2, a vector is prepared comprising a functional Ter site—shown as a darkened circle attached to a darkened diamond. Such a vector may be replicated in a permissive hust, i.e, one that does not express an RTP capable of inhibiting the replication of the plasmid. A desired nucleic acid segment—depicted as an arrow—is to be inserted into the vector. The vector may optionally comprise sites—restriction sites, recombination sites and the like—to facilitate the insertion and/or removal of nucleic acid segments—for example, RS1 and RS2 in Fig. 2. After conducting one or more reactions—recombination reaction and/or digestion and ligation reactions—to insert the segment into the vector a population of molecules is created. In the case of the recombination reaction depicted in Fig. 2, the population includes the desired product as well as unreacted starting vector, and partially reacted vector that includes the insert. Note that the unreacted vector and singly reacted vector both comprise a functional Ter site. When the reaction mixture is transformed into a restrictive host—one that expressed an RTP capable of inhibiting replication of the vector—only those cells that received the desired product—lacking a functional Ter site—can replicate the venter and survive. This is an example of negative selection, i.e., selection against the presence of a Ter site.

With reference to Figs. 3 and 4, positive selection for the presence of an insert, optionally in a desired orientation, is shown. In Fig. 3, a gene of interest is modified to comprise a sequence of a portion of a Ter site— depicted as a darkened circle. A vector is prepared comprising the remaining portion of a Ter site. The remaining portion may be provided as an entire Ter site that can be cleaved in the middle—as shown in Fig. 3—or may be provided as just the retaining sequence. The vector is then cleaved so as to generate a linear vector. When the insert is ligated into the vector it may go in in either orientation. In one orientation, a functional Ter site is generated (plasmid B) and in the other, no Ter site is generated (plasmid A). When the reaction mixture is introduced into host cells expressing an RTP, only those cells that receive a vector that does not contain a functional Ter site (plasmid A) can replicate the vector and grow. This is an example of positive selection for a particular orientation of the insert.

With reference to Fig. 4, a vector is prepared that comprises a functional Ter site that can be cleaved. A gene of interest is ligated into cleaved vector and the reaction mixture is used to transform cells expressing an RTP. Only those cells that receive a vector comprising an insert—and hence lacking a Ter site—can replicate (plasmids A and B) in an RTP+ host. This is an example of positive selection for an insert. Plasmids the self-ligate (plasmid C) will not replicate in an

### Detection Methods

The high affinity of the Ter-binding protein and/or fusion protein comprising a Ter-binding site for the Ter site may advantageously be used to detect molecules comprising a Ter site and/or molecules comprising a Tcr-binding protein. Those skilled in the art will appreciate that a detectable molecule may be attached to a molecule comprising a Ter site, to a molecule comprising a Ter-binding protein, or to both. An example of one detection method is provided in Fig. 8. A nucleic acid of interest (NA) may be attached to a solid support, for example, as in a Northern or Southern blot. A probe comprising a Ter site (black box) and a sequence that specifically hybridizes to the sequence of interest can be hybridized to the target sequence. The probe may optionally comprise a sequence that forms a stem loop structure and/or a hairpin where the Ter site is contained in the double stranded portion of the probe. After hybridization, the complex comprising the probe and the target sequence is contracted with after-binding protein(TBP). The Ter-binding protein may optionally comprise a detection molecule (X), for example, a fluorophore, chromophore, enzyme or the like. Optionally, the Ter-binding protein may not comprise a detection molecule and may instead be detected using an antihody-optionally labeled-to the Ter-binding protein.

The detection methods may be used in a variety of applications including, but not limited to, Southern blots, Northern blots, Western blots, and *in situ* hybridization.

### Purification Methods

The high affinity of the Ter-binding protein and/or fusion protein comprising a Ter-binding site for the Ter site may advantageously be used in a variety of purification methodologies. Molecules comprising nucleic acids comprising a Ter site may be bound to a solid support and used to bind molecules comprising all or a portion of a Ter-binding protein from a solution. Alternatively, molecules comprising all or a portion of a Ter-binding protein may be attached to a solid support and used to bind molecules comprising all or a portion of a Ter site.

In some embodiments, nucleic acids-for example, plasmids-comprising a Ter site may be used as vectors. In embodiments of this type, the presence of the Ter site in the vector may be used to facilitate the manipulation of the nucleic acid. For example, with reference to Figure 6A, a nucleic acid comprising a Ter-site (black box)on a stuffer fragment (wavy line) of a plasmid may be digested with a restriction enzyme at restriction enzyme sites (RE) and un-digested and partially digested plasmid removed from the reaction mixture by being bound through Ter-binding protein to a solid support. Nucleic acid without Ter-sites-correctly digested plasmid in Fig. 6A-are not bond and are thus readily available for further use, such as library construction.

Fig. 6B shows a related aspect in which a vector comprising a Ter site (black box) may contain a sequence of interest-promoter, gene, etc-flanked by restriction and/or recombination sites (RE in Fig. 6B). After the nucleic acid is contacted with the appropriate enzyme-restriction enzyme and/or recombinase-unreacted or partially reacted vector can be removed from solution by contacting the solution with an immobilized protein comprising a Ter-binding site. This facilitates the purification of the product molecule which does not contain a Ter-binding site. The product molecule-i.e., insert-may be subsequently further manipulated as required.

A further embodiment is provided in Fig. 7. In this embodiment, the sequence of interest is amplified or copied from a template comprising a Ter site (black box). The template molecule may be any type of nucleic acid for example, a plasmid or a fragment comprising the sequence of interest. After a sufficient number of copies is prepared, the template molecule may be removed from the reaction mixture by contacting the mixture with an immobilized protein comprising a Ter-binding site (TBP).

Thus, in one aspect, the diclosure describes affinity purification methods comprising (1) providing a support to which one or more Ter-binding proteins are bound, (2) contacting the support with a composition containing molecules or compounds which have binding affinity for Ter-binding protein bound to the support, under conditions which facilitate binding of the molecules or compounds to the Ter-binding protein bound to the support, (3) altering the conditions to facilitate the release of the bound molecules or compounds, and (4) collecting the released molecules or compounds.

In some embodiments, the present diclosure describes methods of purifying molecules that comprise all or a portion of a Ter-binding protein. In one embodiment of this type, a fusion protein comprising a Ter-binding protein can be purified by contacting a solution containing the fusion protein with a compound comprising a nucleic acid having a Ter site, for example a magnetic bead to which is attached an oligonucleotide. After binding, the compound-bead-may be washed and the fusion protein eluted.

Thus, in another aspect, the diclosure describes affinity purification methods comprising (1) providing a support to which nucleic acid molecules comprising at least one Ter site are bound, (2) contacting the support with a composition containing molecules or compounds which have binding affinity for nucleic acid molecules bound to the support, under conditions which facilitate binding of the molecules or compounds to the nucleic acid molecules bound to the support, (3) altering the conditions to facilitate the release of the bound molecules or compounds, and (4) collecting the released molecules or compounds.

### Methods of Manipulating Nucleic Acids

The high affinity of Ter-binding proteins for Ter sites permits various manipulations of nucleic acid molecules that have not been previously possible. For example, with reference to Fig. 9, the affinity of a Ter-binding protein for a Ter site can be used to protect a particular portion of a nucleic acid molecule from, for example, exonuclease digestion. This permits preparation of desired fragments of nucleic acid. In Fig. 9, a fragment of nucleic acid comprising a Ter site (black box) is contacted with a Ter-binding protein (TBP) to form a complex. The fragment is then contacted with an exonuclease, for example a 3' to 5' exonuclease. The fragment is digested until the exonuclease reaches the Ter-binding protein where the digestion is halted. This results in the production of a smaller fragment that terminates at the Ter site. As shown in Fig. 9, the Ter-binding protein may be removed and the overlapping portion of the fragment denatured to produce single strands. The single strands may optionally be converted to double strands by hybridizing a primer-for example, one having the sequence of the Ter site-and extending the primer with a polymerase enzyme and nucleoside triphosphates. The result is to produce a smaller fragment having a defined end.

In some embodiments, the present disclosure describes a method to juxtapose two or more sites in one or more nucleic acid molecules. In its simplest form, a nucleic acid molecule comprising two Ter sites is contacted with a multivalent Ter-binding protein-for example a divalent Ter-binding protein. The multivalent Ter-binding protein binds the nucleic acid at multiple sites thus juxtaposing the sites. In some embodiments, two or more nucleic acids may be juxtaposed. A first nucleic acid comprising a Ter site is contacted with a multivalent Ter-binding protein. The multivalent Ter-binding protein binds the first nucleic acid at the Ter site. The complex of first nucleic acid and Ter-binding protein may optionally be purified from unbound Ter-binding protein and nucleic acid. The complex may then be contacted with a second nucleic acid comprising a Ter site. The multivalent Ter-binding protein then binds the second nucleic acid, thereby juxtaposing the sites. This method may be used to bring sites together for subsequent reactions, for example, ligation and/or recombination reactions.

With reference to Fig. 10, two ends of a linear nucleic acid molecule can be brought together using the present invention. A ds DNA contains a Ter-site at one end "A" and a promoter for an RNA polymerase (indicated by the arrow and T7) near the Ter-site appropriately placed such that DNA/protein interaction and transcription is permitted. The Ter-binding protein (TBP) is functionally associated with the RNA polymerase (T7) that recognizes the promoter, for example, by constructing a fusion protein or chemically coupling a Ter-binding protein to a polymerase. When the Ter-binding protein-RNA polymerase complex is added to the linear ds DNA, the Ter-binding protein binds Ter and RNA polymerase binds the nearby promoter. Addition of nucleotides under certain condition results in transcription by the RNA polymerase which proceeds down the ds DNA toward the other end. The bound Ter-binding protein pulls the "A" end toward the "B" end. The two ends may be annealed or ligated more efficiently when "A" and "B" are in close proximity. Ends of nucleic acid molecules from about 250 base pairs (bp) to 250,000 bp, preferably 1000 - 100,000 bp can be apposed. Polymerases which could be directed to a specific site on a DNA strand can be used such as *E. coli* RNA polymerase holoenzyme, T7 RNA polymerase, or SP6 RNA polymerase, to name a few. In this way, intramolecular joining at the ends of a linear DNA may be increased, and formation of chimeric molecules may be decreased.

In addition to its use in cloning, the ability to juxtapose sites in a nucleic acid molecule may be used in the construction and use of nanodevices. The ability of the Ter-binding protein to hold a specific site on a nucleic acid molecule while another protein-for example, a polymerase-pulls the specific site to some distal point on the nucleic acid molecule can be used to move individual strands of a nanodevice as desired.

With reference to Fig. 11, the present invention can be used to maintain the topology of a nucleic acid. For example, a supercoiled nucleic acid molecule with two Ter sites (black boxes) may be contacted with a divalent Ter-binding protein (TBP-TBP). The Ter-binding protein holds the nucleic acid rigid, maintaining the topology of the region between the two sites. As exemplified in Fig. 11, the nucleic acid may be optionally cleaved to linearize the molecule; however; the region of the molecule between the Ter sites is maintained in a supercoiled form. In some embodiments, a linear molecule with Ter sites at the ends can be supercoiled by first, contacting the molecule with a divalent Ter-binding protein to bind the two sites and then contacting the molecule with a topoisomerase under conditions causing the super coiling of the nucleic acid molecule. This may be useful for transfection of linear fragments, for example, PCR fragments. Fragments may be prepared with primers incorporating Ter sites. After amplification, the fragments may be contacted with a divalent Ter-binding protein and, subsequently, with a topoisomerase and cofactors, resulting in the production of a supercoiled PCR fragment.

With reference to Fig. 12, the present invention may be used to generate a defined overhang in a nucleic acid molecule comprising a Ter site. A first single stranded nucleic acid comprising one strand of a Ter site is contacted with a second nucleic acid comprising the other strand of the Ter site. After the two strands anneal, a Ter-binding protein is added that binds to the reconstituted Ter site. A primer extension reaction using a primer that anneals to the first nucleic acid at a location 3' to the Ter site is conducted. The extension is halted at the Ter-binding protein-Ter complex leaving a nick. The Ter-binding protein and the second nucleic acid are removed leaving a defined overhang.

In some embodiments, the present disclosure describes a method of maintaining a nucleic acid in a duplex under conditions that would normally result in denaturation of the duplex. A nucleic acid comprising one or more Ter sites may be contacted with a Ter-binding protein that recognizes the Ter site. Optionally, the Ter-binding protein may be a thermostable Ter-binding protein. Thermostable Ter-binding proteins may be isolated from thermophilic bacteria or prepared by modifying a Ter-binding protein from a non-thermophilic bacteria. Such modifications include, introducing point mutations in the Ter-binding protein such as introducing cysteine residues to form disulfide bridges, chemically crosslinking the Ter-binding protein using bifunctional crosslinking reagents, cyclizing the Ter-binding protein and the like.

### Kits

In another aspect, the disclosure describes kits which may be used in conjunction with the invention. Kits may comprise one or more containers, which may contain one or more components selected from the group consisting of one or more nucleic acid molecules or vectors, one or more primers, one or more Tcr-binding proteins and/or modified Ter-binding proteins of the invention, supports of the invention, one or more polymerases, one or more reverse transcriptases, one or more recombination proteins (or other enzymes for carrying out the methods of the invention), one or more buffers, one or more detergents, one or more restriction endonucleases, one or more nucleotides, one or more terminating agents (*e.g*., ddNTPs), one or more transfection reagents, pyrophosphatase, one or more proteolytic enzymes and the like.

A wide variety of nucleic acid molecules or vectors of the invention can be used. Further, due to the modularity of the invention, these nucleic acid molecules and vectors can be combined in wide range of ways. Examples of nucleic acid molecules which can be supplied in kits include those that contain all or a portion of one or more Ter sites and, optionally, one or more promoters, signal peptides, enhancers, repressors, selection markers, transcription signals, translation signals, primer hybridization sites (*e.g*., for sequencing or PCR), recombination sites, restriction sites and polylinkers, sites which suppress the termination of translation in the presence of a suppressor tRNA, suppressor tRNA coding sequences, sequences which encode domains and/or regions (*e.g.*, 6 His tag) for the preparation of fusion proteins, origins of replication, telomeres, centromeres, and the like. Similarly, libraries can be supplied in kits. These libraries may be in the form of replicable nucleic acid molecules or they may comprise nucleic acid molecules which are not associated with an origin of replication. As one skilled in the art would recognize, the nucleic acid molecules of libraries, as well as other nucleic acid molecules, which are not associated with an origin of replication either could be inserted into other nucleic acid molecules which have an origin of replication or would be expendable kit components.

Vectors supplied in kits can vary greatly, In most instances, these vectors will contnin an origin of replication, at least one selectable marker, and at least one Ter site and may contain one or more recombination sites. For example, vectors supplied in kits can have four separate recombination sites which allow for insertion of nucleic acid molecules at two different locations. Other attributes of vectors supplied in kits are described elsewhere herein.

Kits can also be supplied with primers. These primers will generally be designed to anneal to molecules having specific nucleotide sequences. For example, these primers can be designed for use in PCR to amplify a particular nucleic acid molecule. Further, primers supplied with kits can be sequencing primers designed to hybridize to vector sequences. Thus, such primers will generally be supplied as part of a kit for sequencing nucleic acid molecules which have been inserted into a vector.

One or more buffers (*e.g*., one, two, three, four, five, eight, ten, fifteen) may be supplied in kits. These buffers may be supplied at a working concentrations or may be supplied in concentrated form and then diluted to the working concentrations. These buffers will often contain salt, metal ions, co-factors, metal ion chelating agents, etc. for the enhancement of activities of the stabilization of either the buffer itself or molecules in the buffer. Further, these buffers may be supplied in dried or aqueous forms. When buffers are supplied in a dried form, they will generally he dissolved in water prior to use. Examples of buffers suitable for use in kits of the invention are set out in the following examples.

Supports suitable for use with the invention (*e.g*., solid supports, semi-solid supports, beads, multi-well tubes, etc., described above in more detail) may also be supplied with kits.

Kits may contain virtually any combination of the components set out above or described elsewhere herein. As one skilled in the art would recognize, the components supplied with kits will vary with the intended use for the kits. Thus, kits may be designed to perform various functions set out in this application and the components of such kits will vary accordingly.

Having now described the present invention in detail, the same will be more clearly understood by reference to the following examples.

### EXAMPLES

### EXAMPLE 1

### Use of RTP/Ter interaction in plasmids

The termination of replication function of the RTP/Ter interaction may be used to select against the presence of Ter sequences in a plasmid. For example, two Ter sequences can be inserted in a particular nucleic acid segment arranged as inverted repeats with the non-permissive side of each Ter-site located proximal to the origin of replication. The replication complex will be unable to replicate the segment of the plasmid in between the Ter-sites. Thus the plasmid will not be replicated and will be lost. Replication may proceed bi-directionally from the origin until the replication complex reaches the termination sequence. In a host cell which produces a functional RTP, replication of the plasmid would be halted at the Ter-sites and the plasmid would not be replicated. In a host cell which does not produce a functional RTP, the plasmid would be replicated.

If desired, the plasmid may comprise one or more additional nucleic acid segments encoding, for example, selectable markers. A selectable marker may be placed at any location on the plasmid including at a location between the Ter-sites that is not replicated in a host that produces a functional RTP, The plasmid can be replicated in a RTP- host strain and will not be replicated in a RTP+ strain. The presence of the plasmid may be selected in a RTP-strain using a suitable negative selection such as an antibiotic, for example, when the selectable marker is an antibiotic resistance conferring gene. Other marker genes include, for example, nutritional markers, heavy metals, halogenated organics, osmotic shock, pH shock, temperature shock, post-segregational killing, allele addition, i.e., ccdB, ccdA, restriction gene sets, and conditional lethal sacB.

Another application of a plasmid containing a Ter site is in recombinational cloning methods. For this method, the plasmid may be equipped with recombination sites (RS 1 and RS2). A plasmid of this type shown in Fig. 2 may be reacted in a recombination reaction with a nucleic acid comprising recombination sites that react with RS1 and RS2. The result would be replacement of the segment containing the Ter-site or sites with a segment from the nucleic acid. Since the resulting molecule would not contain the Ter-site(s), it would be replicated in a RTP+ host cell. Any intermediate molecules resulting from the reaction of only one or the other of RS1 and RS2 would still contain Ter-site(s) and would not be replicated in a RTP+ host.

### EXAMPLE 2

### Attachment of nucleic acids to solid supports.

A nucleic acid with a Ter-site recognized by a RTP or Ter-binding protein can be attached to a solid support via the Ter-binding protein. For example, a Ter-binding protein may be attached to a solid support by covalent linkage. In some embodiments, reactive groups on the Ter-binding protein may be utilized to attach the protein to a solid support (See Fig. 5). For example, a solid support may be prepared comprising a aldehyde functionality to be coupled to an amine present on the protein. Suitable reagents and techniques for conjugation of the Ter-binding protein to a solid support may be found in Hermanson, Bioconjugate Techniques, Academic Press Inc., San Diego, CA, 1996. The binding of Ter-binding protein to Ter-sites may then be used to attach molecules comprising a Ter-site to the solid support.

This methods presents an advantage over standard methods known in the art in that the bound nucleic acids should be more accessible to probes and manipulations because the nucleic acids are attached at one point, not multiple points, as in traditional methods using poly-lysine coatcd glass for example. Target nucleic acids may also be accessible to a Ter-site containing nucleic acid before being introduced into the solid support environment. The Ter-binding protein might then bind a portion or even an entire population of Ter-site-containing nucleic acids. Optionally, interaction of the Ter-site-containing nucleic acid with a target nucleic acid may be necessary for binding to the Ter-binding protein.

### EXAMPLE 3

### Directional cloning of blunt ended fragments.

The present invention provides materials and methods for the directional cloning of blunt ended nucleic acid fragments. The blunt ended fragments may be produced by PCR amplification of a nucleic acid target of interest. In some embodiments, an amplification reaction may be performed in which one of the primers used to amplify the DNA target of interest incorporates a sequence corresponding to a portion of a termination sequence. The product of the amplification reaction will be a blunt ended nucleic acid fragment having a portion of a termination sequence at one end. In order to directionally clone such a fragment, the fragment may be ligated into a vector wherein the vector also comprises a portion of a termination site.

In some preferred embodiments, the portion of the termination site contained by the vector and the portion of the termination site contained by the PCR fragment may combine to form one complete termination site (see Fig. 3). In this situation, the blunt-ended fragment may only be cloned into the vector in one direction. The presence of a complete termination site sequence on the resultant plasmid will make the replication of the plasmid extremely inefficient in the presence of replication terminator protein. Since the replication of the host cell into which the plasmid has been inserted is dependent upon the presence of a plasmid encoding a selectable marker i.e. an antibiotic resistance marker, the replication of host cells containing plasmids in which a complete termination site has been reconstituted will be severely impaired in comparison to those cells in which a termination site was not reconstituted (See Fig. 3).

Thus after ligation two types of vectors will be formed, a vector having a complete termination site sequence and a vector that contains two interrupted portions of a termination site sequence. After transformation two populations of host cells will be formed. One population will comprise a vector containing a complete termination site sequence and the other population will comprise a vector having an interrupted termination site sequence. After growth on a selective media cells containing an interrupted termination sites sequence will grow better than those containing a complete termination sites sequence.

A vector may be constructed so as to introduce a portion of a Ter-site adjacent to a recombination site. In some Preferred embodiments, the portions of the termination site described above may be combine with all or a portion of a recombination site. In embodiments of this type, insertion of the blunt-ended fragment into the vector will result in the production of a vector that comprises a functional recombination site. After identification of colonies containing the vector having the blunt-ended fragment in the proper orientation, the vectors may be further manipulated using recombinational cloning techniques.

Directional cloning provides for the orientation-specific establishment of a DNA segment of interest into a vector. The fact that the orientation of the fragment is known adds significantly to the value of a given clone construction because the orientation of the segment provides information for subsequent reactions such as what sequencing primer to use and where the open reading frame acid is relative to plasmid-bome expression signals.

In situations where positive selection for recombinants is desired, the gene of interest can be cloned into a vector containing a termination sequence wherein the stuffer fragment disrupts the termination sequence. Replacement of the stuffer by the gene of interest disrupts the termination sequence. Non-recombinant vectors without the stuffer will fail to establish upon transformation into cells since re-ligation of the cloning site without an insert recreates a termination site rendering the plasmid nonreplicable (See Fig. 4). Thus, the direction of the cloned insert and selection for the vector containing the insert may be accomplished in the same step by the same sequence element.

### EXAMPLE 4

### Preparation of a selection vector.

In order to demonstrate the utility of the RTP/Ter interaction in selecting a vector having the insert in the desired orientation, a vector was constructed as follows. The pDONR201 (Invitrogen Corporation, Carlsbad, CA) backbone was amplified by PCR using primers that introduced SpeI sites at the core-proximal point of both attL segments. The 5' and 3' sequence of TerB from *E*. *coli* were appended to the 5' and 3' ends of the gene for beta-galactosidase using the polymerase chain reaction (PCR). The primers used in PCR introduced restriction enzyme sites allowing for cloning of the amplicon into the aforementioned plasmid backbone, as well as the subsequent removal of beta-galactosidase from the construct. After excision of the beta galactosidase gene, the resulting linear blunt-ended vector was gel purified (Fig. 3 and Fig. 14). The final vector contained an interrupted TerB site after excision of beta-galactosidase. The 5'-end of the TerB site-the diamond and line in Fig.3-contained nucleotides 1-15 of the TerB sequence in Table 1 while the 3'-end-the circle and line in Fig. 3-contained nucleotides 16-21.

The test insert was constructed using a gene encoding spcctinomycin resistance which was amplified by PCR using primers that appended the 3'-portion TerB element to the 3'-end of the spectinomycin gene. The reverse complement of nucleotides 16-21 of the TerB sequence of Table 1 were added to the 3'-end of the spectinomycin gene. In addition, blunt restriction enzyme sites were introduced distal to the 5' expression signals and 3' inverted Ter sequence. The amplicon was digested with these restriction enzymes to yield a blunt fragment.

Ligation: 5 µl of insert DNA was added to either 1 or 10 µl of vector and ligated in a 20 µl reaction for 2.5 h. at 16°C. In addition, either 1 or 10 µl of vector was subjected to the same reaction conditions without the addition of insert DNA. The reactions were extracted with phenol/chloroform, ethanol precipitated, and reconstituted in 10 µl. One hundred µl of library efficiency DH5a (Invitrogen, Carlsbad, CA) were transformed with each ligation according to the manufacturer's protocol and plated onto LB with kanamycin.

Two distinct colony morphologies apparent, large and small. The results are shown in Table 2.

**TABLE 2**

| | | | | |
|---|---|---|---|---|
| µl insert | 0 | | 5 | |
| µl vector | 1 | 10 | 1 | 10 |
| CFU/100 µl | 0 | 5 | 12 | 95 |

Plasmid DNA was prepared from 8 "no insert" colonies, 121:5 (vector:insert ratio) colonies, and 21 10:5 colonies. Both colony morphologies were picked for DNA preparation. DNA was digested with restriction enzymes diagnostic for presence and orientation of insert. Using colony morphology as predictor, 93% (25/27) had desired orientation. Plasmid yield from 83% (10/12) of undesired orientation was comparatively poor, due either to reduced copy number, lower growth rate, or both. (See Figs. 13A and 13B).

### EXAMPLE 5

### Improving transfection efficiency and targeting of a sequence.

In another aspect, the present invention provides materials and methods for the improvement of transfection efficiency. In some preferred embodiments, nucleic acids comprising one or more Ter-sites may be contacted with a Ter-binding protein in order to improve transfection efficiency and/or expression of a sequence contained on the nucleic acid.. In some embodiments, the Ter-binding protein may be modified to comprise one or more modificalions that improve cellular uptake, cellular localization, stability of the nucleic acid or combinations thereof. In some embodiments, the Ter-binding protein may be modified so as to comprise one or more ligands recognized by one or more cellular receptors. For example, a Ter-binding protein may be derivatized so as to comprise one or more integrin-binding ligands including, but not limited to, proteins or peptides comprising the amino acid sequence arginine-glycine-wpartic acid (RGD). Such protein or peptides may be part of the primary sequence of a fusion protein between such proteins or peptides and afer-binditig protein. In other embodiment, such protein or peptides may be attached to a Ter-binding protein using conventional protein-protein linkers. For example, a protein or peptides comprising an RGD sequence via intrinsic amino groups may be linked using a cross-linking reagent such as glutaraldehyde, In other embodiments, a protein or peptide comprising an RGD sequence may be linked to a Ter-binding protein via other reactive functional moieties such as thiol or hydroxyl moieties. Those skilled in the art will appreciate that the linking of reactive functional moieties is routine in the art of protein chemistry.

In some embodiments of this type, a nucleic acid molecule may comprise more than one Ter-sites. For example, a linear nucleic acid may have a Ter-site on each end of the molecule. The nucleic acid may be contacted with one or more Ter-binding fusion proteins having one or more modifications. In some embodiments, the Ter-binding fusion proteins may comprise two or more different modifications designed to enhance the up take and cellular targeting of the nucleic acid. For example, one Ter-binding fusion protein may be modified to contain a receptor ligand and another to comprise a nuclear localization sequence. The nucleic acid may be contacted with both modified proteins such that one of each type binds to a single nucleic acid molecule. Transfection of the molecule into a cell will be enhanced by the presence of the receptor ligand and expression will be enhanced by the transport of the nucleic acid to the nucleus mediated by the nuclear localization sequence.

### EXAMPLE 6

### Improve gene targeting/knockouts in cells using Ter-binding protein/Ter to protect the ends of linear DNA molecules in vivo.

In some embodiments of the present invention, nucleic acids comprising Ter-sites may be contacted with functional Ter-binding proteins and stable nucleic acid-protein complexes may be formed. The stable complexes may then be transfected into a recipient host cell using conventional technologies. Embodiments of this type may be useful to improve the efficiency of gene targeting/knockouts, e.g., for creating knockouts in cells, e.g., embryonic stem cells. In some preferred embodiments, a nucleic acid may be provided with one or more Ter-sites that may he on each end of the nucleic acid. When molecules of this type are contacted with Ter-binding proteins and/or Ter-binding fusion proteins, the stabile complex may comprise one or more Ter-binding proteins at each end of the nucleic acid. The presence of the Ter-binding protein at the end of the nucleic acid may enhance the stability of the nucleic acid molecule after cellular uptake. A Ter-binding protein for use in embodiments of this type may comprise intracellular targeting sequences, for example nuclear targeting sequences.

In some embodiments, a nucleic acid with two Ter sites may be contacted with a multivalent Ter-binding protein so as to fix the topology of the linear molecule. Optionally, the molecule may he treated to alter the topology by, for example, treating the molecule with one or more topoisomerase enzymes and suitable cofactors.

### EXAMPLE 7

### Using a Ter-binding fusion with a detection molecule for use in the detection of biological molecules.

In some embodiments, the present invention comprises materials and methods for use in the detection of biological molecules. In some embodiments, a Ter-binding protein may comprise a detection molecule. Suitable detection molecules include, but are not limited to, chromophores, fluorophores, enzymes and the like. In some preferred embodiments the detection molecule may be any enzyme whose activity can be measured. Suitable enzymes include, but are not limited to, alkaline phosphatase, bcta-galaetosidase, beta-gtucuronidase and the like. In some embodiments, a Ter-binding protein may comprise multiple detectable moieties which may be the same of different.

In some embodiments, the biological molecule to be detected may be a nucleic acid. In some embodiments, a nucleic acid may be fixed to a solid support such as a filter ad/or an array. In order to detect the nucleic acid of interest, a probe nucleic acid comprising a sequence capable of hybridizing to the nucleic acid of interest may be equipped with a sequence comprising a Ter-site. The Ter-site may he provided in the form of a hairpin molecule or, alternatively, one strand of a Ter-site may be incorporated into the nucleic acid capable of hybridizing to the nucleic acid of interest and a second oligonucleotide having a sequence complementary to the strand of the Ter-site incorporate in a nucleic acid may be provided as a separate molecule. In embodiments of this type, the second oligonucleotide may be provided either before or after the hybridization of the probe nucleic acid to the target nucleic acid. After hybridization of the probe molecule comprising a Ter-site to the target molecule, the Ter-site containing probe molecule may be detected using a Ter-binding protein comprising a detectable portion. This embodiment is exemplified in Fig. 8.

### EXAMPLE 8

### Using Ter-binding protein-coated solid supports.

Solid supports to which one or more Ter-binding proteins have been affixed can be used to purify Ter-site-containing molecules from a mixture. Mixtures may be the result of conducting a desired reaction, e.g. a PCR reaction. The PCR product or the staring template may comprise a Ter site. After completion of the reaction, the Ter-site-containing molecule can be separated from the remainder of the reaction mixture by contacting the mixture with a solid support—for example, magnetic beads—comprising a Ter-binding protein. The remaining components of the mixture can then be washed from the bead and the Ter-site-containing molecule eluted from the solid support. This embodiment can be used to separate a variety of biological molecules from mixtures comprising them. Other embodiments include, but are not limited to, separating vectors from inserts; sequencing products from reaction components, DNA from dNTPs or dNMPs, e.g. PCR reactions or exonuclease reactions; plasmids from minipreps, to name a few.

In some embodiments of the present invention, a Ter-binding protein may be covalently attached to one or more solid supports. Solid supports may be of any form customarily used in the art for example, solid supports may be in the form of filters, fibers, membranes, glass slides, beads, and/or 96 well plates.

To purify the nucleic acid with the Ter-site, the solution comprising the nucleic acid is brought in contact with the Ter-binding protein attached to the solid support to form a complex. The nucleic acids not containing a Ter-site are not bound and can be separated from bound nucleic acid (See Figs. 6A and 6B). This embodiment will be useful in the purification of plasmids from cellular lysates, for example, in a miniprep.

### EXAMPLE 9

### Use of Ter-binding protein/Ter to juxtapose sites in nucleic acid molecules and increase synthesis of product.

In yet another aspect, the present disclosure describes a method for juxtaposing sites in nucleic acid molecules. In one embodiment, a nucleic acid comprising two Ter sites is contacted with a multivalent—i.e., divalent—Ter-binding protein. Each binding site on the nucleic acid molecule binds to a site on the multivalent Ter-binding protein resulting in the juxtaposition of the two sites. The nucleic acid may optionally be subjected to additional manipulations, for example, recombination reactions, endonuclease reactions, ligations and the like.

In another embodiment, the techniques herein described can be used to move sites within a molecule into a desired spatial relationship. For example, the techniques herein described can be used to juxtapose two sites—for example—two ends, "A" and "B" of a linear nucleic acid molecule (See Fig.10). Fig. 10 depicts an embodiment using an enzyme capable of translocating along a nucleic acid molecule. Although Fig. 10 depicts a polymerase enzyme as the translocation enzyme, those skilled in the art will appreciate that other enzymes, for example, helicases may also be used as translocation enzymes.

The dsDNA contains a Ter-site at one end "A" and a promoter for an RNA polymerase near the Ter-site appropriately placed such that DNA/protein interaction and transcription is permitted. The Ter-binding protein is functionally associated with the RNA polymerase that recognizes the promoter, for example, by constructing a fusion protein. When the Ter-binding-RNA polymerase complex is added to the linear ds DNA, Ter-binding protein binds Ter and RNA polymerase binds the nearby promoter. Addition of nucleotides under certain condition results in transcription by the RNA polymerase which proceeds down the ds DNA toward the other end. The bound Ter-binding protein pulls the "A" end toward the "B" end. The two ends may be annealed or ligated more efficiently when "A" and "B" are in close proximity. Ends of nucleic acid molecules from about 250 base pairs (bp) to 250,000 bp, preferably 1000 - 100,000 bp can be apposed. Polymcrascs which could be directed to a specific site on a DNA, strand can be used such as *E*. *coli RNA* polymerase holoenzyme, T7 RNA polymerase, or SP6 RNA polymerase, to name a few, In this way, intramolecular joining at the ends of a linear DNA may be increased, and formation of chimeric molecules may be decreased.

Another aspect of embodiments of this type is an increased rate of re-initiation—and hence synthesis of product—that will be observed as a result of the interaction of the Ter-binding protein-polymerase fusion. After completion of synthesis of a first product, the polymerase portion of the fusion protein may release the template molecule. The Ter-binding portion will not release the template resulting in the polymerase being immediately positioned at the promoter where a subsequent round of initiation and polymerization can begin.

### EXAMPLE 10

### Use of Ter-binding proteins to monitor production of single stranded nucleic acids.

The inability of Ter-binding proteins to bind to single-stranded Ter-sites, can be used to monitor or select for conversion from ds to ss DNA, or vice versa. Monitoring formation of ds DNA can be used to detect formation of ds PCR product, or for real time detection and measurement of formation of double stranded DNA product. For example, amplification of a target sequence may be conducted using a primer that incorporates a Ter sequence. The primer may also comprise a detectable label such as a fluorescent molecule. The amplification may be conducted in the presence of a Ter-binding protein which may optionally comprise a moiety capable of quenching the fluorescence of the detectable label. Since the Ter-binding protein will not bind the primer, the initial fluorescence will not he substantially altered by the Ter-binding protein. As the amplification proceeds, double stranded Tcr sites will be formed and bound by the Ter-binding protein. The presence of the quenching moiety on the Ter-binding protein will result in a reduction of the fluorescence.

In another embodiment, an amplification reaction may be conducted using a Ter-site-containing primer that will contain both a fluorophore and a quencher arranged so that fluorescence is quenched. A Ter-binding protein, modified to comprise an exonuclease, will be added to the amplification reaction. As amplification proceeds forming double stranded Ter sites, the Ter-binding protein will bind the double stranded sites bringing the exonuclease in position to remove the quencher from the double stranded nucleic acid thereby increasing the observed fluorescence as a function of the formation of double stranded nucleic acid.

In another embodiment, an at least partially single stranded nucleic acid comprising at least a portion Ter site may be bound to a solid support. The bound nucleic acid may be contacted with a second nucleic acid that is also at least partially single stranded and the single stranded portion comprises the a sequence complementary to that of the first nucleic acid such that hybridization of the two nucleic acids results in the formation of a Ter-site that may be bound by a Ter-binding protein. The Ter-binding protein may optionally be a modified Ter-binding protein, for examples, The Ter-binding protein may comprise a detectable label.

### EXAMPLE 11

### Use of Ter-binding proteins to produce single stranded nucleic acids.

In yet another aspect, the present invention relates to a method for producing single stranded (ss) DNA from a double-stranded (ds) DNA containing a Ter-site (See Fig. 9). The method includes binding a Ter-binding protein to the Ter-site on the ds DNA, digesting one strand of DNA with an exonuclease, where the bound Ter-binding protein blocks one strand from digestion with the enzyme, and purifying the remaining undigested ss DNA.

In yet another aspect, the present disclosure describes a method for producing a desired fragment. The method includes binding a Ter-binding protein to the Ter-site on a ds DNA, digesting one strand of DNA with an exonuclease, where the bound Ter-binding protein blocks one strand from digestion with the enzyme. Optionally, the remaining undigested ss DNA may be purified. This can be used to produce a single stranded (ss) DNA fragment from a double-stranded (ds) DNA containing a Ter-site (Fig. 9). Optionally, the ssDNA can be converted to dsDNA.

### EXAMPLE 12

### Use of Ter-binding proteins to control topology of a nucleic acid.

In yet another aspect, the present disclosure describes a method for controlling the topology of an nucleic acid molecule. In one aspect, the present disclosure describes a method to maintain superhelicity of linear DNA where the ds, supercoiled DNA contains two Ter-sites one at each end of the segment desired to remain supercoiled after linearization (Fig. 11). A multivalent Ter-binding protein, such as a bivalent Ter-binding protein, is added such that both Ter-sites can be bound and result in insulating one topological domain from another such that one domain can rotate independently of the other. The bivalent Ter-binding proteins can be made by cloning, with or without linkers, direct repents of the open reading frame encoding a Ter-binding protein or by crosslinking the two molecules, for example. Once the DNA fragment is linearized, the unlinearized domain contained by Ter-sites remains supercoiled until one of the Ter-binding proteins is released. This method is useful for reactions where supercoiling is beneficial.

In another aspect, a linear nucleic acid molecule with two Ter sites can be supercoiled between the two Ter-sites by contacting the linear nucleic acid with a divalent Ter-binding protein to form a complex and contacting the complex, with one or more topoisomerase enzymes under conditions resulting in the supercoiling of the molecule.

### EXAMPLE 13

### Using Ter-binding protein/Ter interaction to stop a polymerization reaction at a defined site on a nucleic acid molecule,

The presence of a Ter site in a nucleic acid molecule can be used to generate less than full length products in a polymerization reaction, i.e., a PCR reaction or a transcription reaction. For example, a nucleic acid comprising a promoter, for example a T7 promoter, and a Ter site arranged such that transcription from the promoter is directed toward the Ter site, may be contacted with a T7 polymerase and appropriate cofactors. When the nucleic acid has a Ter-binding protein bound to the Ter site, the transcription will proceed until the polymerase is halted by the Ter-binding protein resulting in the production of transcripts of a defined length.

In another aspect, this method may be used to generate a double stranded fragment with a "sticky end" for ease in cloning using PCR. Referring to Fig. 12, an oligonucleotide #1 is generated comprising a single stranded exploitable sequence A, a top strand of duplex Ter site ter' and a segment capable of annealing to the template. Oligonucleotide #2 comprises a bottom strand of duplex Ter site which hybridizes to tear' of oligonucleotide #1.

When oligonucleotide #1 and oligonucleotide #2 are annealed, a complete double stranded Ter site is generated which is attached to a sequence which hybridizes to the desired template. A thermostable Ter-binding protein which recognizes the Ter site is allowed to bind such that the replication fork encountering the complex from the right is halted.

The PCR reaction is started by introducing the template. During PCR, the polymerase is halted at the right side of Ter-binding protein/Ter complex resulting in a nick at that locus.

After PCR, the double stranded DNA is isolated, deproteinized, resulting in the loss of oligonucleotide #2, to generate the desired overhang.

### EXAMPLE 14

### Methods for detecting biological molecules.

In another aspect, the present disclosure describes methods for detecting a biological molecule, comprising the steps of contacting a biological molecule with a reagent, the reagent comprising a nucleic acid portion preferably containing at least one Ter-site and a portion which forms a specific complex with the biological molecule, contacting the complex with a Ter-binding protein fused to a detection molecule, wherein the Ter-binding protein binds to the nucleic acid portions of the reagent, and detecting the detection molecule, wherein the presence of the detection molecule correlates to the presence of the biological molecule. In some embodiments, the detection molecule may be selected from a group consisting of chromophores, fluorophores, enzymes, and epitopes.

## Claims

1. A solid support comprising:
at least one nucleic acid molecule that comprises all or a portion of a Ter site, wherein the nucleic acid molecule is directly attached to the support; and
at least one fusion protein having one or more Ter-binding portions and one or more additional polypeptide portions;
wherein the fusion protein is indirectly attached to the support via attachment to the nucleic acid molecule through interaction between the nucleic acid molecule Ter site or portion thereof and the fusion protein Ter-binding portion.

2. A solid support according to claim 1, wherein the support is a non-biological material.

3. A solid support according to claim 1, wherein the nucleic acid molecule is capable of forming a stem-loop or hairpin

4. A solid support according to claim 3, wherein a duplex portion of a stem-loop or hairpin comprises a Ter-site.

5. A solid support according to claim 1, wherein the one or more additional polypeptide portions comprise a detection molecule.

6. A solid support according to claim 5, wherein the detection molecule is a fluorescent moiety, a chromophore, an enzyme, a hapten or an epitope recognized by an antibody.

7. A solid support according to claim 1, wherein the one or more additional polypeptide portions are fused to the N-terminus or to the C-terminus of the Ter-binding protein.

8. A solid support according to claim 1, wherein the Ter-binding portion comprises all or a portion of Tus.

9. A solid support according to claim 1, wherein the support comprises one or more silicon, biochips, nitrocellulose, diazocellulose, glass, polystyrene, polyvinylchlorine, polypropylene, polyvinylidenedifluoride (PVDF), dextran, sepharose, agar, starch, nylon, polymerized Langmuir Blodgett film, functionalized glass, Si, Ge, GaAs, GaP, SiO₂, SiN₄, modified silicon, (poly)tetrafluoroethylene, (poly)vinylidenedifluoride, polystyrene, or polycarbonate.

10. A solid support according to claim 1, wherein the support is a multi-well plate, a glass slide, a membrane, a filter, a sheet, a frit, a column, beads, or a microarray.

11. A solid support according to claim 1, wherein the support is a microarray.

12. A method for attaching a Ter-binding protein and/or fusion protein comprising a Ter-binding site to a solid support, comprising:
directly attaching a nucleic acid molecule comprising one or more Ter-sequences to a solid support; and
contacting the nucleic acid molecule with a Ter-binding protein and/or a fusion protein comprising a Ter-binding site;
wherein the Ter-binding protein and/or fusion protein binds to said nucleic acid molecule through interaction at one or more Ter-sites.

13. A method for attaching a nucleic acid to a solid support, comprising:
attaching one or more Ter-binding proteins to a solid support; and
contacting the Ter-binding protein with a first nucleic acid, said nucleic acid comprising a Ter-site.

14. A method according to claim 12 or 13, wherein the Ter-binding protein is a Tus protein or RTP.

15. A method according to claim 13, further comprising contacting the first nucleic acid with a second nucleic acid.

## Patentansprüche

1. Fester Träger, welcher umfasst:
mindestens ein Nukleinsäure-Molekül, das eine vollständige oder einen Teilbereich einer Ter-Stelle umfasst, wobei das Nukleinsäure-Molekül direkt mit dem Träger verbunden ist, und
mindestens ein Fusionsprotein mit einem oder mehreren Ter-Bindungsbereichen und einem oder mehreren zusätzlichen Polypetid-Bereichen,
wobei das Fusionsprotein indirekt, durch Verbindung mit dem Nukleinsäure-Molekül mittels Wechselwirkung zwischen der Ter-Stelle des Nukleinsäure-Moleküls oder eines Teilbereichs davon und dem Ter-Bindungsbereich des Fusionsproteins, mit dem Träger verbunden ist.

2. Fester Träger nach Anspruch 1, wobei der Träger ein nicht-biologisches Material ist.

3. Fester Träger nach Anspruch 1, wobei das Nukleinsäure-Molekül zur Bildung einer Stamm-Schleife oder eines Hairpin fähig ist.

4. Fester Träger nach Anspruch 3, wobei ein Duplex-Bereich einer Stamm-Schleife oder eines Hairpin eine Ter-Stelle umfasst.

5. Fester Träger nach Anspruch 1, wobei der eine oder die mehreren zusätzlichen Polypetid-Bereich(e) ein Detektionsmolekül umfassen.

6. Fester Träger nach Anspruch 5, wobei das Detektionsmolekül ein fluoreszierender Rest, ein Chromophor, ein Enzym, ein Hapten oder ein durch einen Antikörper erkanntes Epitop ist.

7. Fester Träger nach Anspruch 1, wobei der eine oder die mehreren zusätzlichen Polypetid-Bereich(e) mit dem N-Terminus oder dem C-Terminus fusioniert sind.

8. Fester Träger nach Anspruch 1, wobei der Ter-Bindungsbereich alles oder einen Teilbereich von Tus umfasst.

9. Fester Träger nach Anspruch 1, wobei der Träger eines oder mehrere von Silikon, Biochips, Nitrozellulose, Diazozellulose, Glas, Polystyren, Polyvinylchlorid, Polypropylen, Polyvinylidenedifluorid (PVDF), Dextran, Sepharose, Agar, Stärke, Nylon, polymerisierter Langmuir-Blodgett-Schicht, funktionalisiertem Glas, Si, Ge, GaAs, GaP, SiO₂, SiN₄, modifiziertem Silikon, (Poly-)Tetrafluorethylen, (Poly-)Vinylidenedifluorid, Polystyren oder Polycarbonat umfasst.

10. Fester Träger nach Anspruch 1, wobei der Träger eine Multiwell-Platte, ein Glas-Objektträger, eine Membran, ein Filter, ein Blatt, eine Fritte, eine Säule, Beads oder ein Microarray umfasst.

11. Fester Träger nach Anspruch 1, wobei der Träger ein Microarray ist.

12. Verfahren zum Verbinden eines Ter-Bindungsproteins und/oder eines Fusionsproteins, das eine Ter-Bindungsstelle umfasst, mit einem festen Träger, welches umfasst:
direktes Verbinden eines Nukleinsäure-Moleküls, das eine oder mehrere Ter-Sequenzen umfasst, mit einem festen Träger, und
in Kontakt Bringen des Nukleinsäure-Moleküls mit einem Ter-Bindungsprotein und/oder einem Fusionsprotein, das eine Ter-Bindungsstelle umfasst,
wobei das Ter-Bindungsprotein und/oder das Fusionsprotein durch Wechselwirkung an einer oder mehreren Ter-Stelle(n) an das Nukleinsäure-Molekül bindet.

13. Verfahren zum Verbinden einer Nukleinsäure mit einem festen Träger, welches umfasst:
Verbinden eines oder mehrerer Ter-Bindungsproteine mit einem festen Träger, und
in Kontakt Bringen des Ter-Bindungsproteins mit einer ersten Nukleinsäure, wobei die Nukleinsäure eine Ter-Stelle umfasst.

14. Verfahren nach Anspruch 12 oder 13, wobei das Ter-Bindungsprotein ein Tus-Protein oder RTP ist.

15. Verfahren nach Anspruch 13, welches ferner das in Kontakt Bringen der ersten Nukleinsäure mit einer zweiten Nukleinsäure umfasst.

## Revendications

1. Support solide comprenant :
au moins une molécule d'acide nucléique qui comprend la totalité ou une partie d'un site Ter, où la molécule d'acide nucléique est fixée directement au support ; et
au moins une protéine de fusion comportant une ou plusieurs parties de liaison de Ter et une ou plusieurs parties polypeptidiques supplémentaires ;
où la protéine de fusion est fixée indirectement au support par l'intermédiaire d'une fixation à la molécule d'acide nucléique par l'intermédiaire d'une interaction entre le site Ter de la molécule d'acide nucléique ou une partie de celui-ci et la partie de liaison de Ter de la protéine de fusion.

2. Support solide selon la revendication 1, où le support est un matériau non biologique.

3. Support solide selon la revendication 1, où la molécule d'acide nucléique est capable de former une tige-boucle ou une épingle à cheveux.

4. Support solide selon la revendication 3, où une partie duplex d'une tige-boucle ou d'une épingle à cheveux comprend un site Ter.

5. Support solide selon la revendication 1, où les une ou plusieurs parties polypeptidiques supplémentaires comprennent une molécule de détection.

6. Support solide selon la revendication 5, où la molécule de détection est un fragment fluorescent, un chromophore, une enzyme, un haptène ou un épitope reconnu par un anticorps.

7. Support solide selon la revendication 1, où les une ou plusieurs parties polypeptidiques supplémentaires sont fusionnées à l'extrémité N-terminale ou à l'extrémité C-terminale de la protéine de liaison de Ter.

8. Support solide selon la revendication 1, où la partie de liaison de Ter comprend la totalité ou une partie de Tus.

9. Support solide selon la revendication 1, où le support comprend un ou plusieurs du silicium, des biopuces, de la nitrocellulose, de la diazocellulose, du verre, du polystyrène, du polyvinylchloré, du polypropylène, du polyvinylidène fluorure (PVDF), du dextrane, du sépharose, de la gélose, de l'amidon, du nylon, du film de Langmuir-Blodgett polymérisé, du verre fonctionnalisé, Si, Ge, GaAs, GaP, SiO₂, SiN₄, du silicium modifié, du (poly)tétrafluoroéthylène, du (poly)vinylidène difluorure, du polystyrène ou du polycarbonate.

10. Support solide selon la revendication 1, où le support est une plaque à plusieurs puits, une lame de verre, une membrane, un filtre, une feuille, un fritté, une colonne, des billes ou une micropuce.

11. Support solide selon la revendication 1, où le support est une micropuce.

12. Procédé de fixation d'une protéine de liaison de Ter et/ou d'une protéine de fusion comprenant un site de liaison de Ter à un support solide, comprenant :
la fixation directe d'une molécule d'acide nucléique comprenant une ou plusieurs séquences Ter à un support solide ; et
la mise en contact de la molécule d'acide nucléique avec une protéine de liaison de Ter et/ou une protéine de fusion comprenant un site de liaison de Ter ;
où la protéine de liaison de Ter et/ou la protéine de fusion se lie à ladite molécule d'acide nucléique par l'intermédiaire d'une interaction au niveau d'un ou de plusieurs sites Ter.

13. Procédé de fixation d'un acide nucléique à un support solide, comprenant :
la fixation d'une ou de plusieurs protéines de liaison de Ter à un support solide ; et
la mise en contact de la protéine de liaison de Ter avec un premier acide nucléique, ledit acide nucléique comprenant un site Ter.

14. Procédé selon la revendication 12 ou 13, où la protéine de liaison de Ter est une protéine Tus ou RTP.

15. Procédé selon la revendication 13, comprenant en outre la mise en contact du premier acide nucléique avec un second acide nucléique.


### SEQUENCE LISTING

<110> Invitrogen Corporation
<120> Compositions and Methods for Molecular Biology
<130> 0942.523PC01
<150> 60/266,846
   <151> 2001-02-07
<160> 25
<170> PatentIn version 3.1
<210> 1
   <211> 23
   <212> DNA
   <213> Escherichia coli
<400> 1
   aattagtatg ttgtaactaa agt 23
<210> 2
   <211> 23
   <212> DNA
   <213> Escherichia coli
<400> 2
   aataagtatg ttgtaactaa agt 23
<210> 3
   <211> 23
   <212> DNA
   <213> Escherichia coli
<400> 3
   atataggatg ttgtaactaa tat 23
<210> 4
   <211> 23
   <212> DNA
   <213> Escherichia coli
<400> 4
   cattagtatg ttgtaactaa atg 23
<210> 5
   <211> 21
   <212> DNA
   <213> Escherichia coli
<400> 5
   ttaaagtatg ttgtaactaa g 21
<210> 6
   <211> 23
   <212> DNA
   <213> Escherichia coli
<400> 6
   ccttcgtatg ttgtaacgac gat 23
<210> 7
   <211> 23
   <212> DNA
   <213> Escherichia coli
<400> 7
   gatgagtatg ttgtaactaa cta 23
<210> 8
   <211> 23
   <212> DNA
   <213> Salmonella typhimurium
<400> 8
   attaagtatg ttgtaactaa agc 23
<210> 9
   <211> 23
   <212> DNA
   <213> Salmonella typhimurium
<400> 9
   gatgagtatg ttgtaactaa atg 23
<210> 10
   <211> 23
   <212> DNA
   <213> Artificial Sequence
<220>
   <223> Replication terminator sequence R6KterR1
<400> 10
   ctcttgtgtg ttgtaactaa atc 23
<210> 11
   <211> 23
   <212> DNA
   <213> Artificial Sequence
<220>
   <223> Replication termination sequence R6KterR2
<400> 11
   ctattgagtg ttgtaactac tag 23
<210> 12
   <211> 23
   <212> DNA
   <213> Artificial Sequence
<220>
   <223> Replication termination sequence R100 TerR1
<400> 12
   attatgaatg ttgtaactac ttc 23
<210> 13
   <211> 23
   <212> DNA
   <213> Artificial Sequence
<220>
   <223> Replication termination sequence R100TerR2
<400> 13
   tgtctgagtg ttgtaactaa agc 23
<210> 14
   <211> 23
   <212> DNA
   <213> Artificial Sequence
<220>
   <223> Replication termination sequence R1TerR1
<400> 14
   attatgaatg ttgtaactac atc 23
<210> 15
   <211> 23
   <212> DNA
   <213> Artificial Sequence
<220>
   <223> Replication termination sequence R1TerR2
<400> 15
   tttttgtgtg ttgtaactaa att 23
<210> 16
   <211> 23
   <212> DNA
   <213> Artificial Sequence
<220>
   <223> Replication termination sequence RepFICTerR1
<400> 16
   attatgaatg ttgtaactac att 23
<210> 17
   <211> 23
   <212> DNA
   <213> Artificial Sequence
<220>
   <223> Replication termination sequence St90kbTer
<400> 17
   attttggatg ttgtaactat ttg 23
<210> 18
   <211> 30
   <212> DNA
   <213> Bacillus atrophaeus
<400> 18
   gaactaaata aactatgtac caaatgttca 30
<210> 19
   <211> 30
   <212> DNA
   <213> Bacillus atrophaeus
<400> 19
   taactgaaaa cactatgtac taaatattca 30
<210> 20
   <211> 30
   <212> DNA
   <213> Bacillus mojavensis
<400> 20
   gaacaaaaca aactatgtac caaatgttca 30
<210> 21
   <211> 30
   <212> DNA
   <213> Bacillus mojavensis
<400> 21
   aaactgagaa tactatgtac taaatattca 30
<210> 22
   <211> 30
   <212> DNA
   <213> Bacillus vallismortis
<400> 22
   atactaaaaa tatgatgtac taaatattca 30
<210> 23
   <211> 30
   <212> DNA
   <213> Bacillus amyloliquefaciens
<400> 23
   taacaaatta ttccatgtac taaatattct 30
<210> 24
   <211> 30
   <212> DNA
   <213> Bacillus subtilis 168
<400> 24
   gaactaatta aactatgtac taaattttca 30
<210> 25
   <211> 30
   <212> DNA
   <213> Bacillus subtilis 168
<400> 25
   atactaattg atccatgtac taaattttca 30